# EUROPÄISCHE PATENTSCHRIFT

(11) **EP 2 342 174 B1**
(45) Veröffentlichungstag und Bekanntmachung des Hinweises auf die Patenterteilung: **27.03.2013**
(21) Anmeldenummer: 09778778.2
(22) Anmeldetag: 30.09.2009
(51) Int. Cl.: C07C 209/68, C07C 213/08, C07C 215/46, C07D 311/80

(54) **VERFAHREN ZUR HERSTELLUNG VON 3-AMINOBIPHENYLEN**
METHOD FOR PRODUCING 3-AMINOBIPHENYLENE
PROCEDE DE PREPARATION DE 3-AMINOBIPHENYLENE

(30) Priorität: 30.09.2008 DE 102008049675
(43) Veröffentlichungstag der Anmeldung: 13.07.2011
(73) Patentinhaber: Technische Universität München, 80333 München (DE)
(72) Erfinder: HEINRICH, Markus, 86316 Friedberg (DE); WETZEL, Alexander, 89423 a. d. Donau (DE)
(74) Vertreter: Forstmeyer, Dietmar
(86) Internationale Anmeldenummer: PCT/EP2009/007020
(87) Internationale Veröffentlichungsnummer: WO 2010/037531

(56) Entgegenhaltungen:
- EP-A1- 2 072 498
- DE-C1- 10 032 134
- LESLIE M. WERBEL. ET.AL.: "Synthesis, Antimalarial Activity, and Quantitative Structure-Activity-relationship of Tebuquine and a Series of Related 5-[(7-Chloro-4-quinolinyl)amino]-3-[(alkyl amino)methyl][1,1'biphenyl]-2-ols and N-Oxides" J. MED. CHEM., Bd. 29, Nr. 6, 1986, Seiten 924-939, XP002569990
- PAUL M. O'NEILL ET.AL.: "Synthesis, Antimalarial Activity, and Molecular Modeling of Tebuquine Analogues" J. MED. CHEM., Bd. 40, 1997, Seiten 437-448, XP002569910 in der Anmeldung erwähnt
- CEPANEC ET AL: "Copper(I)-catalysed homo-coupling of aryldiazonium salts: synthesis of symmetrical biaryls" TETRAHEDRON, ELSEVIER SCIENCE PUBLISHERS, AMSTERDAM, NL, Bd. 63, Nr. 25, 19. Mai 2007 (2007-05-19), Seiten 5614-5621, XP022084936 ISSN: 0040-4020
- ROBINSON ET AL: "Palladium-catalyzed homocoupling of arenediazonium salts: an operationally simple synthesis of symmetrical biaryls" TETRAHEDRON LETTERS, ELSEVIER, AMSTERDAM, NL, Bd. 48, Nr. 43, 26. September 2007 (2007-09-26), Seiten 7687-7690, XP022272168 ISSN: 0040-4039

## Beschreibung

Die vorliegende Erfindung betrifft ein Verfahren zur Herstellung von substituierten 3-Aminobiphenylen.

Zur milden und effizienten Synthese von Biarylverbindungen steht heute eine breite Palette metallorganischer Methoden zur Verfügung:
Übersichtsartikel zu metallorganischen Biarylsynthesen: M. Beller, C. Bolm, Transition Metals for Organic Synthesis, 2nd ed., Wiley-VCH, Weinheim, 2004; A. de Meijere, F. Diederich, Metal-Catalyzed Cross-Coupling Reactions, 2nd ed., Wiley-VCH, Weinheim, 2004.
Neueste Entwicklungen auf dem Gebiet der Biarylsynthese: T. Dohi, M. Ito, K. Morimoto, M. Iwata, Y. Kita, Angew. Chem. 2008, 120, 1321-1324, Angew. Chem. Int. Ed. 2008, 47, 1301-1304; M. Amatore, C. Gosmini, Angew. Chem. 2008, 120, 2119-2122, Angew. Chem. Int. Ed. 2008, 47, 2089-2092; L. J. Gooßen, N. Rodriguez, K. Gooßen, Angew. Chem. 2008, 120, 3144-3164, Angew. Chem. Int. Ed. 2008, 47, 3100-3120.
Biarylkupplung über CH-Aktivierung: G. Dyker, Handbook of C-H Transformations, Wiley-VCH, Weinheim, 2005; L. Ackermann, Top. Organomet. Chem. 2007, 24, 35-60; T. Vogler, A. Studer, Org. Lett. 2008, 10, 129-131; L. Ackermann, R. Vicente, A. Althammer, Org. Lett. 2008, 10, 2299-2302.

Allerdings sind organometallische Methoden auch mit einigen Nachteilen behaftet. So wird ihre Attraktivität durch hohe Kosten der Ausgangsmaterialien, insbesondere bei Palladiumkatalysierten Umsetzungen, mangelnde Umweltverträglichkeit, wie im Falle des Nickels, und geringe Ausgereiftheit, insbesondere bei der Katalyse mit Cobalt- und Eisenverbindungen, gemindert.

Verglichen mit dieser Vielfalt an Transformationen sind Additionsreaktionen von Arylradikalen an aromatische Substrate bisher nur selten zum Einsatz gekommen. Übersichtsartikel zur radikalischen Biarylsynthese: A. Studer, M. Brossart in Radicals in Organic Synthesis, Eds. P. Renaud, M. P. Sibi, 1st ed., Wiley-VCH, Weinheim, 2001, Vol. 2, 62-80; W. R. Bowman, J. M. D. Storey, Chem. Soc. Rev. 2007, 36, 1803-1822; J. Fossey, D. Lefort, J. Sorba, Free Radicals in Organic Chemistry, Wiley, Chichester, 1995, 167-180 Intramolekulare radikalische Biarylkupplungen werden dabei vor allem durch die vergleichsweise langsamen Additionsgeschwindigkeiten von Arylradikalen an gängige Substrate wie substituierte Benzole kompliziert, was in der Folge Nebenreaktionen begünstigt (J. C. Scaiano, L. C. Stewart, J. Am. Chem. Soc. 1983, 105, 3609-3614). Erfolgreiche Biarylsynthesen sind deshalb häufig an spezielle Bedingungen gebunden, wobei man das Substrat als Lösungsmittel einsetzt (A. Nunez, A. Sanchez, C. Burgos, J. Alvarez-Builla, Tetrahedron 2004, 60, 6217-6224, P.T.F. McLoughlin, M. A. Clyne, F. Aldabbagh, Tetrahedron 2004, 60, 8065-8071) oder die Reaktion intramolekular durchführt wird (M. L. Bennasar, T. Roca, F. Ferrando, Tetrahedron Lett. 2004, 45, 5605-5609). Biarylkupplungsreaktionen mit Aryldiazoniumsalzen als Radikalvorläufern sind als Gomberg-Bachmann (M. Gomberg, W. E. Bachmann, J. Am. Chem. Soc. 1924, 46, 2339-2343, J. R. Beadle, S. H. Korzeniowski, D. E. Rosenberg, B. J. Garcia-Slanga, G. W. Gokel, J. Org. Chem. 1984, 49, 1594-1603) oder Pschorr-Reaktionen (R. Pschorr, Chem. Ber. 1896, 29, 496-501) bekannt.

In diesen Fällen besteht die Schwierigkeit stets darin, dass für Generierung der Arylradikal ein Reduktionsmittel benötigt wird (C. Galli, Chem. Rev. 1988, 88, 765-792), während oxidative Bedingungen für die Rearomatisierung des Cyclohexadienyl-Intermediats erforderlich sind (D. P. Curran, A. L Keller, J. Am. Chem. Soc. 2006, 128, 13706-13707).

Weiterer Stand der Technik ist in folgenden Dokumenten genannt:
DE 100 32 134;
Cepanec et al.: "Copper(I)-catalysed homo-coupling of aryldiazonium salts: synthesis of symmetrical biaryls", Tetrahedron 63 (2007) 5614-5621;
Robinson et al.: "Palladium-catalyzed homocoupling of arenediazonium salts: an operationally simple synthesis of symmetrical biaryls", Tetrahedron Letters 48 (2007) 7687-7690.

Einzelne Beispiele für Additionsreaktionen von Arylradikalen an Anilinderivate sind zwar seit langem bekannt, doch hat diese Synthesemethode für Biarylamine bisher keine Bedeutung erlangt. Allan und Muzik (Chem. Abstr. 1953, 8705) beschreiben beispielsweise die Umsetzung des Diazoniumsalzes von *para*-Nitroanilin mit Benzidin sowie *N,N,N',N'-*Tetramethylbenzidin. Während das Tetramethylderivat die radikalische Biarylkupplung eingeht, reagiert das unsubstituierte Benzidin nach einem nicht-radikalischen Mechanismus zum entsprechenden Triazen. Nachteil der bekannten radikalischen Biarylkupplungen ist, dass sie meist wenig selektiv verlaufen.

Der vorliegenden Erfindung lag die Aufgabe zu Grunde, ein einfach durchzuführendes Verfahren zur Herstellung von kernsubstituiertem 3-Aminobiphenylen und Derivaten davon bereitzustellen. Dieses Verfahren sollte zudem kostengünstig durchführbar sein und auf selektiven Umsetzungen beruhen. Funktionalisierte Biphenyl-Verbindungen sind insbesondere als Pharmazeutika und Pflanzenschutzmittel sowie als Vorstufen solcher Wirkstoffe von großem Interesse.

Die Aufgabe wird durch die im Folgenden näher beschriebenen Verfahren zur Herstellung von 3-Aminobiphenylen und Derivaten davon gelöst.

Die vorliegende Erfindung betrifft ein Verfahren zur Herstellung einer Verbindungen der Formel 3, 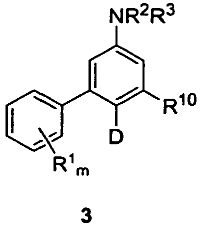 dadurch gekennzeichnet, dass eine Verbindung der Formel **1** 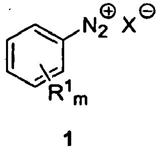 mit einer Verbindung der Formel **2** 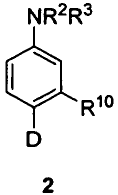 umgesetzt wird, wobei
m für 0, 1, 2, 3, 4 oder 5 steht;
jedes R¹ jeweils unabhängig für Halogen, Alkyl, Haloalkyl, Hydroxyalkyl, Alkoxy, Haloalkoxy, Alkylthio, Cycloalkyl, Haloalkylthio, Alkenyl, Alkinyl, Nitro, Cyano, -SO₃R⁵, -SO₂NH₂, -SO₂NHR⁴, -SO₂NR⁴R⁵, - COOR⁴, -CONHR⁴, -CONR⁴R⁵, -COR⁴, -OCOR⁴, -NR⁴COR⁵, - NR⁴SO₂R⁵, Alkylcarbonyl, Haloalkylcarbonyl, Alkenylcarbonyl, Alkoxycarbonyl, Haloalkoxycarbonyl, Alkenyloxycarbonyl, Alkylsulfonyl, Haloalkylsulfonyl, Alkylimino, Aryl, Aryloxy, Arylcarbonyl, Arylalkyl, Arylmethoxycarbonyl, Arylalkylimino, oder Heteroaryl steht, wobei die Arylgruppe gegebenenfalls 1, 2, 3 oder 4 Substituenten trägt, die ausgewählt sind unter Halogen, Alkyl, Haloalkyl, Alkoxy oder Haloalkoxy;
oder zwei Reste R¹ zusammen eine cyclische Gruppe z.B. der Formel -O-(CR¹¹R¹²)ₜ-O-bilden, wobei t für 1, 2 oder 3 steht, R¹¹ für Wasserstoff, Alkyl oder Haloalkyl steht, R¹² für Wasserstoff, Alkyl, Haloalkyl steht oder ein R¹¹ und ein R¹² zusammen ein nichtaromatisches Ringsystem bilden, welches 5, 6 oder 7 Kohlenstoffatome enthält;
D für Hydroxy, Alkoxy, Thioalkyl, Haloalkyloxy, Alkylcarbonyloxy, Haloalkylcarbonyloxy, -O-(CH₂)ₙ-NR⁴R⁵, -O-(CH₂)ₙNHCOR⁴, -O-(CH₂)ₙ-NHC(O)OR⁴, -O-(CH₂)ₙ-COOR⁴, -O-(CH₂)ₙ-CONHR⁴, -O-(CH₂)ₙ-CONR⁴R⁵, -O-(CH₂)ₙ-SO₃R⁴, -O- (CH₂)ₙ-CN oder Aryloxy steht, wobei die Arylgruppe gegebenenfalls 1, 2, 3, 4 oder 5 Substituenten trägt, die ausgewählt sind unter Halogen, Alkyl, Haloalkyl, Alkoxy, Haloalkoxy, Hydroxy, oder Alkoxycarbonyl; oder
in Verbindung 3 einer der Reste R¹ und D zusammen eine Gruppe der Formel -O- oder -C(O)-O- bilden (wobei dieser Rest R¹ vorzugsweise in ortho Position zu dem Phenylring steht, der D trägt und wobei die CO Gruppe an der Stelle gebunden ist, die R¹ trägt und das O Atom an der Stelle gebunden ist, die D trägt);
X für Halogenid, Sulfat, Tetrafluoroborat, Acetat, Trifluoracetat, Hexafluorophosphat, Hexafluoroantimonat, das Anion eines aromatischen 1,2-Dicarbonsäureimids oder das Anion eines aromatischen 1,2-Disulfonsäureimids steht;
R² jeweils unabhängig für Wasserstoff, Alkyl, Hydroxyalkyl, Cycloalkyl, Aryl, Heteroaryl, -(CH₂)ₙ-NR⁴R⁵, -(CH₂)ₙ-NHCOR⁴, -(CH₂)ₙ-NHC(O)OR⁴, - (CH₂)ₙ-COOR⁴, -(CH₂)ₙ-CONHR⁴, -(CH₂)ₙ-CONR⁴R⁵, -(CH₂)ₙ-SO₃R⁴, - (CH₂)ₙ-CN oder Haloalkyl steht;
R³ jeweils unabhängig für Wasserstoff, Alkyl, Hydroxylalkyl, Cycloalkyl, Aryl, Heteroaryl, -(CH₂)ₙ-NR⁴R⁵, -(CH₂)ₙ-NHCOR⁴, -(CH₂)ₙ-NHC(O)OR⁴, - (CH₂)ₙ-COOR⁴, -(CH₂)ₙ-CONHR⁴, -(CH₂)ₙ-CONR⁴R⁵, -(CH₂)ₙ-SO₃R⁴, - (CH₂)ₙ-CN oder Haloalkyl steht;
n jeweils unabhängig für 1, 2, 3, 4, oder 5 steht;
R⁴ jeweils unabhängig für Wasserstoff, Alkyl, Hydroxylalkyl, Cycloalkyl, Aryl, Heteroaryl oder Haloalkyl steht;
R⁵ jeweils unabhängig für Wasserstoff, Alkyl, Hydroxyalkyl, Cycloalkyl, Aryl, Heteroaryl oder Haloalkyl steht;
R¹⁰ jeweils unabhängig für Wasserstoff, Alkyl, Halogen, Hydroxyalkyl, Cycloalkyl, Aryl, Heteroaryl, -(CH₂)_{q}-NR⁴R⁵, -(CH₂)_{q}-NHCOR⁴, - (CH₂)_{q}-NHC(O)OR⁴, -(CH₂)_{q}-COOR⁴, -(CH₂)_{q}-CONHR⁴, -(CH₂)_{q}-CONR⁴R⁵, -(CH₂)_{q}-SO₃R⁴, -(CH₂)_{q}-CN oder Haloalkyl steht; und
q jeweils unabhängig für 1, 2, 3, 4, oder 5 steht.

Die vorliegende Erfindung betrifft somit ein Verfahren zur Herstellung von 3-Aminobiphenylen der Struktur **3** durch Umsetzung von substituierten Aryldiazoniumsalzen der Struktur **1** mit *para*-substituierten Anilinen der Struktur **2**:

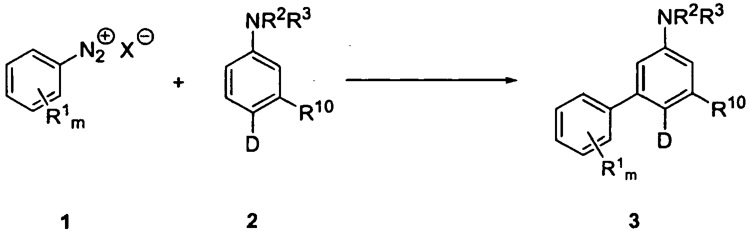

Die Verbindungen der Struktur **3** können beispielsweise als Zwischenprodukte zur Herstellung von biologisch aktiven Verbindungen verwendet werden.

Ein Beispiel ist der Antimalariawirkstoff Tebuquin **4** (P. M. O'Neill, D. J. Willock, S. R. Hawley, P. G. Bray, R. C. Storr, S. A. Ward, B. K. Park, J. Med. Chem. 1997, 40, 437-448), dessen Synthese (ausgehend vom Biarylkupplungsprodukt) im Rahmen der Beispiele beschrieben wird.

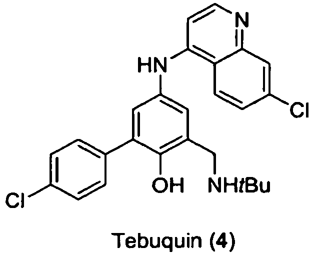

Speziell substituierte 5-Aminobiphenyl-2-ole der Struktur **5** können nach dem hier beschriebenen Verfahren kostengünstig hergestellt werden. Metallorganische Kreuzkupplungsreaktionen, die zu Verbindungen **5** führen, müssen demgegenüber mit vergleichsweise schwierig (nicht kommerziell) zugänglichen Ausgangsmaterialien durchgeführt werden.

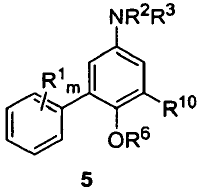

Hierbei steht
R⁶ für Wasserstoff, Alkyl, Hydroxyalkyl, Cycloalkyl, Alkylcarbonyl, Haloalkylcarbonyl, Aryl, Heteroaryl, -(CH₂)_{w}-NR⁴R⁵, -(CH₂)_{w}-NHCOR⁴, -(CH₂)_{w}-NC(O)OR⁴, -(CH₂)_{w}-COOR⁴, -(CH₂)_{w}-CONHR⁴, -(CH₂)_{w}-CONR⁴R⁵, -(CH₂)_{w}-SO₃R⁴, -(CH₂)_{w}-CN oder Haloalkyl; und
w jeweils unabhängig für 1, 2, 3, 4, oder 5; die übrigen Reste sowie m sind wie oben definiert.

Beispiele von biologisch aktiven Verbindungen, die 5-Aminobiphenyl-2-ole **5** als Strukturelement aufweisen, und die deshalb mittels der radikalischen Biarylsynthese zugänglich gemacht werden können, sind in den folgenden Literaturstellen beschrieben:
a) Y. Yamaguchi, T. Yanase, S. Muto, A. Itai, *Preparation of N-phenyloxamidic acid derivatives as inhibitors of plasminogen activator inhibitor-1 (PAI-1).* PCT Int. Appl. **2008**, WO 2008044731.
b) V. L. Nienaber, J. Greer, C. Abad-Zapatero, D. W. Norbeck, *Ligand screening and design by x-ray crystallography.* U.S. **2001**, Cont.-in-part ofU.S. Ser. No. 36,184. US 6297021.
c) S. S. Hadida Ruah, M. T. Miller, B. Bear, J. McCartney, P. D. J. Grootenhuis, *Cycloalkylcarboxamides and related compounds as modulators of ATP-binding cassette transporters and their preparation, pharmaceutical compositions and use in the treatment of diseases.* U.S. Pat. Appl. Publ. **2008**, Cont.-in-part of U.S. Ser. No. 647,092, US 2008009524.
d) R. Palin, C. A. Gray, *Preparation of biaryl benzimidazoleacetamides as vanilloid receptor (TRPV1) inhibitors for the treatment of pain and respiratory disorders.* PCT Int. Appl. **2007**, WO2007054480.
e) R. Palin, C. A. Gray, *Preparation of benzimidazolylbiarylacetamides as vanilloid TRVP1 receptor antagonists.* U.S. Pat. Appl. Publ. **2007**, US 2007112034.
f) T. Luebbers, M. Boehringer, L. Gobbi, M. Hennig, D. Hunziker, B. Kuhn, B. Loeffler, P. Mattei, R. Narquizian, J.-U. Peters, Y. Ruff, H.-P. Wessel, P. Wyss, 1,3-Disubstituted 4-aminopiperidines as useful tools in the optimization of the 2-aminobenzo[a]quinolizine dipeptidyl peptidase IV inhibitors. Bioorganic & Medicinal Chemistry Letters 2007, 17(11), 2966-2970.
g) C. J. Wheelhouse, A. J. F. Thomas, D. J. Bushnell, J. Lumley, J. I. Salter, M. C. Carter, N. Mathews, C. J. Pilkington, R. M. Angell, *Preparation of heterocyclyl biphenylcarboxamides for treatment of hepatitis C virus (HCV) infection.* PCT Int. Appl. **2007**, WO 2007031791.
h) W. Mederski, U. Emde, G. Bamickel, F. Zenke, H. Greiner, F. Stieber, *Preparation of N-pyridinylphenyl-3-4-diaminocyclobut-3-ene-1,2-diones as CHK1, CHK2, and*/*or SGK kinase inhibitors for treating cancer.* PCT Int. Appl. **2007**, WO 2007014607.
i) L. Clary, J.-G. Boiteau, *Preparation of new diaromatics, particularly ureidobiphenylpropanoic acid derivatives, as PPAR activators, and their use in cosmetic and pharmaceutical compositions.* PCT Int. Appl. **2006**, WO 2006018326.
j) A. Ali, J. M. Napolitano, Q. Deng, Z. Lu, P. J. Sinclair, G. E. Taylor, C. F. Thompson, N. Quraishi, C. J. Smith, J. A. Hunt, A. A. Dowst, Y.-H. Chen, H. Li, *Preparation of cholesteryl ester transfer protein (CETP) inhibitors.* PCT Int. Appl. **2006**, WO 2006014413.
k) A. D. Brown, C. A. L. Lane, R. A. Lewthwaite, *Preparation of 2-(6-aminopyridin-3-yl)-2-hydroxyethylamines as 2 adrenoceptor agonists.* PCT Int. Appl. **2004**, WO 2004108676.
l) L. Schwink, S. Stengelin, M. Gossel, T. Boehme, G. Hessler, G. Rosse, A. Walser, *Preparation of 1,3-dihydro-1,3-diphenyl-2H-imidazol-2-ones and related compounds as MCH receptor modulators for the treatment of obesity.* PCT Int. Appl. **2004**, WO 2004011438.
m) Y. Amemiya, K. Wakabayashi, S. Takaishi, C. Fukuda, *Preparation of nitrophenylcarboxamide derivatives as peroxisome proliferator-activated receptor (PPAR) modulators.* PCT Int. Appl. **2001**, WO 2001083427.
n) P. C. Astles, T. J. Brown, P. Cox, F. Halley, P. M. Lockey, C. McCarthy, I. M. McLay, T. N. Majid, A. D. Morley et al. New nonpeptidic C5a receptor antagonists. Bioorganic & Medicinal Chemistry Letters 1997, 7(7), 907-912.
o) K. Akasaka, M. Yonaga, A. Kajiwara, K. Higurashi, K. Ueno, S. Nagato, M. Komatsu, N. Kitazawa, M. Ueno et al. *Preparation of (piperazinylalkyl)biphenyl derivatives as antagonists of dopamine 2 andlor serotonin 2 receptors.* Eur. Pat. Appl. **1995**, EP 675118.
p) G.B. Barlin, C. Jiravinyu, G. A. Butcher, B. Kotecka, K. Rieckmann, The in vitro and in vivo antimalarial activity of some Mannich bases derived from 4-(7'-trifluoromethyl-1'5'-naphthyridin-4'-ylamino)phenol, 2-(7'-trifluoromethyl-quinolin-4'ylamino)phenol, and 4'-chloro-5-(7"-triflluoromethylquinolin-4"-ylamino)biphenyl-2-ols. Annals of Tropical Medicine & Parasitology 1992, 86(4), 323-331.
q) N. Bayraktar, M. Kajbaf, S. D. Jatoe, J. W. Gorrod, The oxidation of isomeric amino and acetamidobiphenyls by rat hepatic microsomal preparations. Archives of Toxicology 1987, 60(1-3), 91-92.

Weiterhin wird ein Verfahren zur Synthese von Verbindungen der Struktur **7** beschrieben, dadurch gekennzeichnet, dass in einem ersten Schritt eine Verbindung der Formel **8** mit einer Verbindung der Formel **9** zu einer Verbindung der Formel **6** umgesetzt wird und in einem weiteren Schritt die Verbindung der Formel **6** in eine Verbindung der Formel **7** überführt wird: 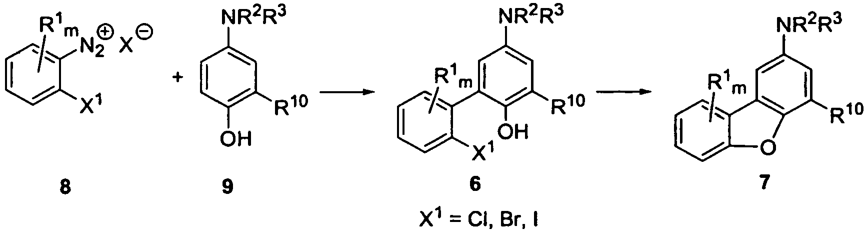 wobei alle Reste wie oben definiert sind und m für 1, 2, 3 oder 4 steht.

Bevorzugte Reaktionsbedingungen für die Überführung der Verbindung der Formel **6** in eine Verbindung der Formel **7** sind Erhitzen in Gegenwart von Basen, wie Natriumhydroxid, Natrium-*tert*-butylat oder Natriumacetat, oder Palladium-katalysierte Umsetzungen in Gegenwart der o.g. Basen.

Substituierte Dibenzofuran-2-ylamine **7** sind wichtige Vorstufen biologisch aktiver Verbindungen. Beispiele für biologisch aktive Verbindungen sind in folgenden Literaturstellen beschrieben:
a) A. Torrens, J. Mas, A. Port, J. A. Castrillo, O. Sanfeliu, X. Guitart, A. Dordal, G. Romero, M. A. Fisas, E. Sanchez, E. Hemandez, P. Perez, R. Perez, H. Buschmann, J. Med. Chem. 2005, 48, 2080-2092.
b) R. A. Smith, A. Bhargava, C. Browe, J. Chen, J. Dumas, H. Hatoum-Mokdad, R. Romero, Bioorg. Med. Chem. Lett. 2001, 11, 2951-2954.
c) B. Jung, F. Himmelsbach, G. Pohl, *New pharmaceutical compositions for treatment of respiratory and gastrointestinal disorders.* PCT Int. Appl. **2008**, WO 2008049842.

Die Herstellung von divers funktionalisierten Dibenzofuranen **7** wurde von Schimmelschmidt in J. L. Ann. Chem. 1950, 566, 184-204 und von Kawaguchi et al. in J. Org. Chem. 2007, 72, 5119 - 5128 beschrieben.

Des weiteren wird ein Verfahren zur Herstellung von Verbindungen der Formel **11** beschrieben, dadurch gekennzeichnet, dass eine Verbindung der Formel **10** mit einer Verbindung der Formel **2** umgesetzt wird, 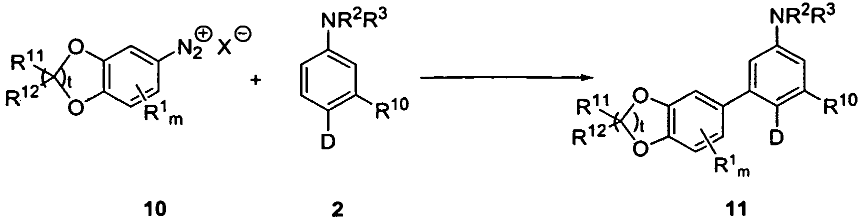 wobei
t für 1, 2 oder 3 steht;
R¹¹ für Wasserstoff, Alkyl oder Haloalkyl steht;
R¹² für Wasserstoff, Alkyl, Haloalkyl steht oder
ein R¹¹ und ein R¹² zusammen ein nichtaromatisches Ringsystem bilden, welches 5, 6 oder 7 Kohlenstoffatome enthält;
m für 1, 2 oder 3 steht
und alle weiteren Reste wie oben definiert sind.

Beispiele für biologisch aktive Verbindungen **11** sind in folgenden Literaturstellen beschrieben:
a) J. Green, M. J. Arnost, A. Pierce, *Preparation of pyrazolone derivatives as inhibitors of GSK-3, Aurora-2 and CDK-2.* PCT Int. Appl. **2003**, WO 2003011287, Chem. Abstr. 138:170229.
b) L. Chassot, H.-J. Braun, *2-Hydroxy-5-aminobiphenyl derivatives for oxidation hair dyeing compositions.* Ger. **2001**, DE 10032134 , Chem Abstr. 136:42512.

Des weiteren wird ein Verfahren zur Herstellung von Verbindungen der Formel **14** beschrieben, dadurch gekennzeichnet, dass eine Verbindung der Formel **12** mit einer Verbindung der Formel **13** umgesetzt wird. 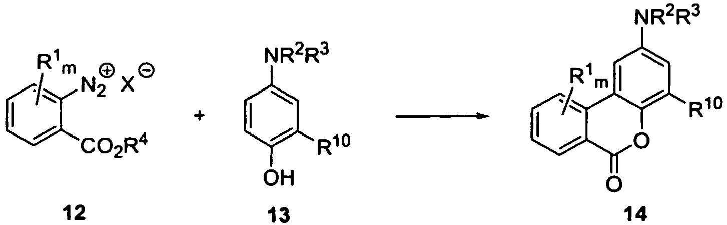 wobei alle Reste wie oben definiert sind und m für 1, 2, 3 oder 4 steht.

Die Anwendung von Verbindungen der Formel **14** als biologisch aktive Verbindungen und Biosensoren ist in den folgenden Literaturstellen beschrieben:
a) D. I. Sherris, *Synthesis of benzo[c]chromen-6-one derivatives and analogs for treatment of diseases characterized by cellular proliferation and angiogenesis.* U.S. Pat. Appl. Publ. **2007**, Cont.-in-part of U.S. Ser. No. 412,618, US 2007197567.
b) D. I. Sherris, *Compositions of benzo(c)chromen-6-ones for treatment of skin diseases characterized by cellular proliferation and angiogenesis.* U.S. Pat. Appl. Publ. **2006**, US 2006257337.
c) E. M. Bellott, D. Bu, J. J. Childs, C. R. Lambert, H. A. Nienaber, S. J. Shi, Z. Wang, J. J. Workman Jr., A. R. Zelenchuk, *SMMR (small molecule metabolite reporters) for use as in vivo glucose biosensors.* PCT Int. Appl. **2005**, WO 2005065241.
d) N. Aoyama, H. Takenaka, A. Miike, Akira. *Coumarin derivatives for quantitative determination of peroxidation-active substances by chemiluminescence analysis.* PCT Int. Appl. **1993**, WO 9315219.
e) H. Liu, M. Santostefano, Y. Lu, S. Safe, 6-Substituted 3,4-benzocoumarins: a new structural class of inducers and inhibitors of CYP1A1-dependent activity. Archives of Biochemistry and Biophysics 1993, 306(1), 223-31.

Des weiteren wird ein Verfahren zur Herstellung einer Verbindungen der Struktur **18** beschrieben, dadurch gekennzeichnet dass in einem ersten Schritt eine Verbindung der Formel **1** mit einer Verbindung der Formel **2**, wobei R² und R³ = H, zu einer Verbindung der Formel **3**, wobei R² und R³ = H, umgesetzt wird und in einem weiteren Schritt die Verbindung der Formel **3** in eine Verbindung der Formel **17** überführt wird. Die Herstellung von Verbindungen der Struktur **17** gelingt unter den unten genauer ausgeführten Bedingungen zur Diazotierung aromatischer Amine.

In einem weiteren Schritt werden die Verbindungen der Formel **17** nach literaturbekannten Verfahren in Verbindungen der Struktur **18** überführt. Die reduktive Deaminierung zu **18** (R¹³ = H) kann unter verschiedensten Reaktionsbedingungen durchgeführt werden (A. Wetzel, V. Ehrhardt, M. R. Heinrich, Angew. Chem. Int. Ed. 2008, 47, 9130-9133) Halogenverbindungen., Thiole und Thioether **18** (R¹³ = -SH, -SAlkyl, -SHaloalkyl, -SAryl, - SHeteroaryl, Halogen, Cyano) sind unter Sandmeyer-Bedingungen zugänglich (F. Minisci, F. Fontana, E. Vismara, Gazz. Chim. Ital. 1993, 123, 9-18). Umsetzungen von **17** beispielsweise unter den Bedingungen der Heck-Reaktion liefern alkenylsubstituierte Verbindungen der Struktur **18** (R¹³ = -CR¹⁴=CR¹⁵-COOH, -CR¹⁴=CR¹⁵-COOAlkyl, -CR¹⁴=CR¹⁵-COOHaloalkyl, -CR¹⁴=CR¹⁵-CN) (S. Sengupta, S. Bhattacharya, J. Chem. Soc. Perkin Trans 1, 1993, 17, 1943.1944). Reaktionsbedingungen für die Umsetzungen von Vebindungen der Struktur 17 mit aromatischen Substraten unter Erhalt von arylierten Biphenylen **18** (R¹³ = Aryl, Heteroaryl) werden in der Einleitung und in den folgenden Ausführungen vielfach beschrieben.

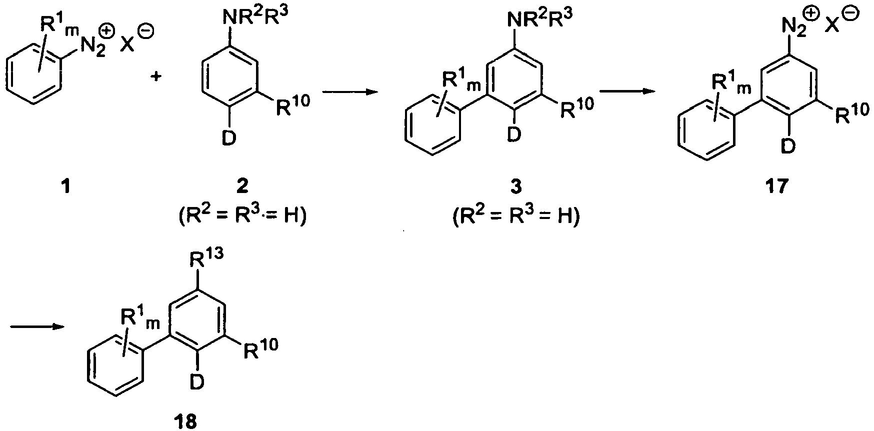

Dabei stehen
R¹³ für Wasserstoff, -SH, -S-Alkyl, -S-Haloalkyl, -S-Aryl, -S-Heteroaryl, Halogen, Cyano, Aryl, Heteroaryl, -CR¹⁴=CR¹⁵-COOH, -CR¹⁴=CR¹⁵-COO-Alkyl, -CR¹⁴=CR¹⁵-COO-Haloalkyl, -CR¹⁴=CR¹⁵-CN, -CR¹⁴=CR¹⁵-Aryl, -CR¹⁴=CR¹⁵-Heteroaryl,
R¹⁴ für Wasserstoff, Alkyl, Haloalkyl
R¹⁵ für Wasserstoff, Alkyl, Haloalkyl, -COOH, -COO-Alykl, -COO-Haloalkyl, -Cyano, Aryl, Heteroaryl, -NHC(O)-Alkyl; -NHC(O)O-Alkyl; und
m für 1, 2, 3, 4 oder 5.
Weiterhin sind alle weiteren Reste wie oben definiert.

Anwendungen von Verbindungen der Strukturformel **18** sind in folgenden Literaturstellen beschrieben:
a) P. C.Astles, T. J. Brown, P. Cox, F. Halley, P. M. Lockey, C. McCarthy, I. M. McLay, T. N. Majid, A. D. Morley, B. Porter, A. J. Ratcliffe, R. J. A. Walsh, Non-peptidic C5a receptor antagonists, Bioorg. Med. Chem. Lett. 1997, 7, 907-912.
b) H. Inagaki, H. Tsuruoka, S. A. Lesley, G. Spraggon, J. A. Ellman, Characterization and optimization of selective, nonpeptidic inhibitors of Cathepsin S with an unprecedented binding mode, J. Med. Chem. 2007, 50, 2693-2699.
c) P.-A. Crassous, C. Cardinaletti, A. Carrieri, B. Bruni, M. Di Vaira, F. Gentili, F. Ghelfi, M. Gianella, H. Paris, A. Piergentili, W. Quaglia, S.Schaak, C. Vesprini, M. Pigini, α2-Adrenoreceptors Profile Modulation: (R)-(+)-m-Nitrobiphenyline, a new and efficient α2C-subtypeslective agonist, J. Med. Chem. 2007, 50, 3964-3968.

Durch weitere Umsetzung kann Verbindung **5** (mit R² und R³ = H), in eine Verbindung der Formel **20** überführt werden. Die Herstellung von Verbindungen der Struktur **19** aus Verbindungen der Struktur **5** gelingt unter den unten genauer ausgeführten Bedingungen zur Diazotierung aromatischer Amine.

In einem weiteren Schritt werden die Verbindungen der Formel **19** nach literaturbekannten Verfahren (siehe oben) in Verbindungen der Struktur **20** überführt. 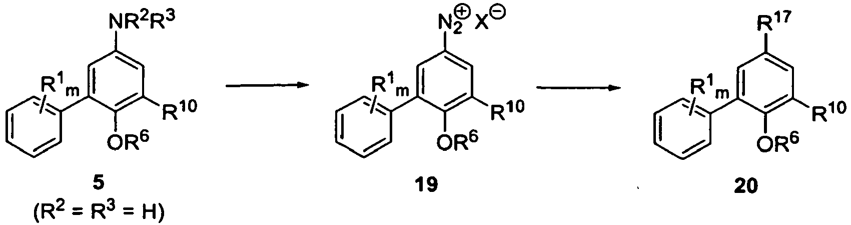 wobei
R¹⁷ für Wasserstoff, -SH, -S-Alkyl, -S-Haloalkyl, -S-Aryl, -S-Heteroaryl, Halogen, Cyano, Aryl, Heteroaryl, -CR¹⁸=CR¹⁹-COOH, -CR¹⁸=CR¹⁹-COO-Alkyl, -CR¹⁸=CR¹⁹-COO-Haloalkyl, -CR¹⁸=CR¹⁹-CN, -CR¹⁸=CR¹⁹-Aryl, -CR¹⁸=CR¹⁹-Heteroaryl,
R¹⁸ für Wasserstoff, Alkyl, Haloalkyl; und
R¹⁹ für Wasserstoff, Alkyl, Haloalkyl, -COOH, -COO-Alykl, -COO-Haloalkyl, -Cyano, Aryl, Heteroaryl, -NHC(O)-Alkyl; -NHC(O)O-Alkyl steht;
und wobei m und alle anderen Reste wie oben definiert sind.

Durch weitere Umsetzung kann die Verbindung **7**, (mit R² und R³ = H), in eine Verbindung der Formel **23** überführt werden. Die Herstellung von Verbindungen der Struktur **22** aus Verbindungen der Struktur **7** gelingt unter den unten genauer ausgeführten Bedingungen zur Diazotierung aromatischer Amine.

In einem weiteren Schritt werden die Verbindungen der Formel **22** nach literaturbekannten Verfahren (siehe oben) in Verbindungen der Struktur **23** überführt. 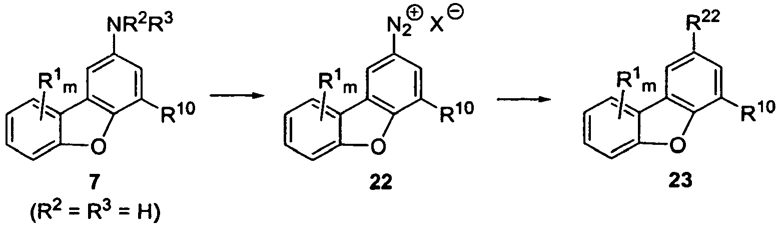 wobei
R²² für Wasserstoff, -SH, -S-Alkyl, -S-Haloalkyl, -S-Aryl, -S-Heteroaryl, Halogen, Cyano, Aryl, Heteroaryl, -CR²³=CR²⁴-COOH, -CR²³=CR²⁴-COO-Alkyl, -CR²³=CR²⁴-COO-Haloalkyl, -CR²³=CR²⁴-CN, -CR²³=CR²⁴-Aryl, -CR²³=CR²⁴-Heteroaryl,
R²³ für Wasserstoff, Alkyl, Haloalkyl
R²⁴ für Wasserstoff, Alkyl, Haloalkyl, -COOH, -COO-Alykl, -COO-Haloalkyl, -Cyano, Aryl, Heteroaryl, -NHC(O)-Alkyl; -NHC(O)O-Alkyl; und
wobei m für 1, 2, 3 oder 4 steht und alle anderen Reste wie oben definiert sind.

Durch weitere Umsetzung kann Verbindung **11** (mit R² und R³ = H), in eine Verbindung der Formel **29** überführt werden. Die Herstellung von Verbindungen der Struktur **28** aus Verbindungen der Struktur **11** gelingt unter den unten genauer ausgeführten Bedingungen zur Diazotierung aromatischer Amine.

In einem weiteren Schritt werden die Verbindungen der Formel **28** nach literaturbekannten Verfahren (siehe oben) in Verbindungen der Struktur **29** überführt. 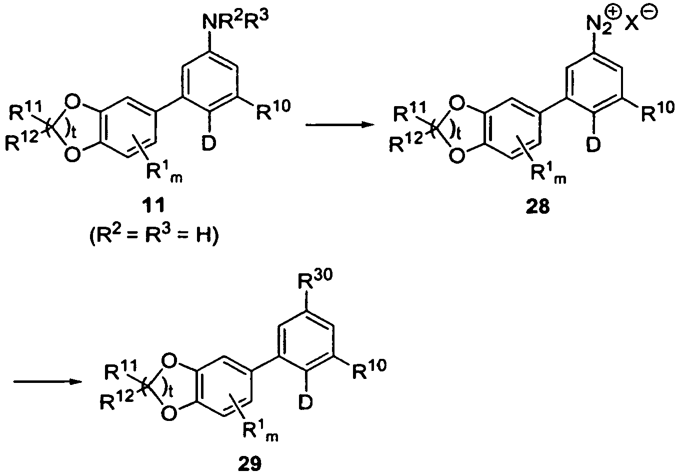 wobei
R³⁰ für Wasserstoff, -SH, -S-Alkyl, -S-Haloalkyl, -S-Aryl, -S-Heteroaryl, Halogen, Cyano, Aryl, Heteroaryl, -CR³¹=CR³²-COOH, -CR³¹=CR³²-COO-Alkyl, -CR³¹=CR³²-COO-Haloalkyl, -CR³¹=CR³²-CN, -CR³¹=CR³²-Aryl, -CR³¹=CR³²-Heteroaryl,
R³¹ für Wasserstoff, Alkyl, Haloalkyl
R³² für Wasserstoff, Alkyl, Haloalkyl, -COOH, -COO-Alykl, -COO-Haloalkyl, -Cyano, Aryl, Heteroaryl, -NHC(O)-Alkyl; -NHC(O)O-Alkyl; und
m für 1, 2 oder 3 steht und
wobei alle anderen Reste wie oben definiert sind.

Durch weitere Umsetzung kann die Verbindung **14** (mit R² und R³ = H), in eine Verbindung der Formel **32** überführt werden. Die Herstellung von Verbindungen der Struktur **31** aus Verbindungen der Struktur **14** gelingt unter den unten genauer ausgeführten Bedingungen zur Diazotierung aromatischer Amine.

In einem weiteren Schritt werden die Verbindungen der Formel **31** nach literaturbekannten Verfahren (siehe oben) in Verbindungen der Struktur **32** überführt. 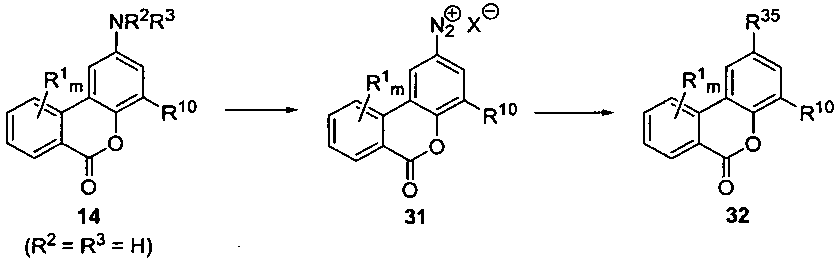 wobei
R³⁵ für Wasserstoff, -SH, -S-Alkyl, -S-Haloalkyl, -S-Aryl, -S-Heteroaryl, Halogen, Cyano, Aryl, Heteroaryl, -CR³⁶=CR³⁷-COOH, -CR³⁶=CR³⁷-COO-Alkyl, -CR³⁶=CR³⁷-COO-Haloalkyl, -CR³⁶=CR³⁷-CN, -CR³⁶=CR³⁷-Aryl, -CR³⁶=CR³⁷-Heteroaryl,
R³⁶ für Wasserstoff, Alkyl, Haloalkyl
R³⁷ für Wasserstoff, Alkyl, Haloalkyl, -COOH, -COO-Alykl, -COO-Haloalkyl, -Cyano, Aryl, Heteroaryl, -NHC(O)-Alkyl; -NHC(O)O-Alkyl; und
m für 1, 2, 3 oder 4 steht und
wobei alle anderen Reste wie oben definiert sind.

Im Rahmen der vorliegenden Erfindung haben die generisch verwendeten Begriffe die folgenden Bedeutungen:
Das Präfix Cₓ-C_{y} bezeichnet im jeweiligen Fall die Anzahl möglicher Kohlenstoffatome.
Der Begriff "Halogen" bezeichnet jeweils Fluor, Brom, Chlor oder Iod, speziell Fluor, Chlor oder Brom, besonders bevorzugt Fluor oder Chlor.
Der Begriff "Alkyl" bezeichnet einen linearen oder verzweigten Alkylrest, umfassend 1 bis 20 Kohlenstoffatome, wie Methyl, Ethyl, Propyl, 1-Methylethyl (Isopropyl), Butyl, 1-Methylpropyl (sec-Butyl), 2-Methylpropyl (Isobutyl), 1,1-Dimethylethyl (tert-Butyl), Pentyl, Hexyl, Heptyl, Octyl, 2-Ethylhexyl, Nonyl, Decyl oder 2-Propylheptyl und Stellungsisomere davon, bevorzugt Methyl, Ethyl oder Propyl.
Der Begriff "Haloalkyl", wie hierin und in den Haloalkyleinheiten von Haloalkoxy verwendet, beschreibt geradkettige oder verzweigte Alkylgruppen mit 1 bis 10 Kohlenstoffatomen, wobei die Wasserstoffatome dieser Gruppen teilweise oder vollständig durch Halogenatome ersetzt sind. Beispiele hierfür sind Chlormethyl, Brommethyl, Dichlormethyl, Trichlormethyl, Fluormethyl, Difluormethyl, Trifluormethyl, Chlorofluormethyl, Dichlorfluormethyl, Chlordifluormethyl, 1-Chlorethyl, 1-Bromethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2 Dichlor-2-fluorethyl, 2,2,2-Trichlorethyl, Pentafluorethyl, 3,3,3-Trifluorprop-1-yl, 1,1,1-Trifluorprop-2-yl, 3,3,3-Trichlorprop-1-yl, Heptafluorisopropyl, 1-Chlorbutyl, 2-Chlorbutyl, 3-Chlorbutyl, 4-Chlorbutyl, 1-Fluorbutyl, 2-Fluorbutyl, 3-Fluorbutyl, 4-Fluorbutyl und dergleichen, bevorzugt Fluormethyl, 2-Fluorethyl, oder Trifluormethyl.
Der Begriff "Alkenyl" bezeichnet einen einfach ungesättigten (Doppelbindung), linearen oder verzweigten aliphatischen Rest mit 2 oder 3 bis 8 Kohlenstoffatomen. Beispiele hierfür sind Propen-1-yl, Propen-2-yl (Allyl), But-1-en-1-yl, But-1-en-2-yl, But-1-en-3-yl, But-1-en-4-yl, But-2-en-1-yl, But-2-en-2-yl, But-2-en-4-yl, 2-Methylprop-1-en-1-yl, 2-Methylprop-2-en-1-yl und dergleichen, bevorzugt Propenyl oder But-1-en-4-yl.
Der Begriff "Alkinyl" bezeichnet einen linearen oder verzweigten aliphatischen Rest mit 2 oder 3 bis 8 Kohlenstoffatomen und mindestens einer Dreifachbindung.
Der Begriff "Cycloalkyl" bezeichnet einen gesättigten alicyclischen Rest mit 3 bis 10 Kohlenstoffatomen als Ringglieder. Beispiele sind Cyclopropyl, Cyclobutyl, Cyclopentyl, Cyclohexyl, Cycloheptyl, Cyclooctyl, Cyclononyl und Cyclodecyl. Die Cycloalkylreste können 1, 2 oder 3 Substituenten tragen, die ausgewählt sind unter Alkyl, Alkoxy oder Halogen, bevorzugt Cyclopropyl, Cyclobutyl, Cyclopentyl oder Cyclohexyl.
Der Begriff "cyclische Gruppe" bezeichnet eine gesättigte oder ungesättigte (z.B. aromatische) cyclische Gruppe mit 3 bis 10 Ringatomen, ausgewählt aus O, S, N und Kohlenstoffatomen, vorzugsweise mit 5, 6 oder 7 Ringatomen.
Der Begriff "Alkoxy" (oder "Alkyloxy") bezeichnet geradkettige oder verzweigte gesättigte Alkylgruppen, umfassend 1 bis 10 Kohlenstoffatome, die über ein Sauerstoffatom gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele für Alkoxy sind Methoxy, Ethoxy, n-Propoxy, 1-Methylethoxy (Isopropoxy), n-Butoxy, 1-Methylpropoxy (sec-Butoxy), 2-Methylpropoxy (Isobutoxy) und 1,1-Dimethylethoxy (tert-Butoxy), bevorzugt Methoxy, Ethoxy, n-Propoxy, oder -OCH₂-*cyclo*-Pentyl.
Der Begriff "Haloalkoxy" (oder "Haloalkyloxy") beschreibt geradkettige oder verzweigte gesättigte Haloalkylgruppen, umfassend 1 bis 10 Kohlenstoffatome, die über ein Sauerstoffatom gebunden sind. Beispiele hierfür sind Chlormethoxy, Brommethoxy, Dichlormethoxy, Trichlormethoxy, Fluormethoxy, Difluormethoxy, Trifluormethoxy, Chlorofluormethoxy, Dichlorfluormethoxy, Chlordifluormethoxy, 1-Chlorethoxy, 1-Bromethoxy, 1-Fluorethoxy, 2-Fluorethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, 1,1,2,2-Tetrafluorethoxy, 1-Chlor-1,2,2-trifluorethoxy, Pentafluorethoxy, 3,3,3-Trifluorprop-1-oxy, 1,1,1-Trifluorprop-2-oxy, 3,3,3-Trichlorprop-1-oxy, 1-Chlorbutoxy, 2-Chlorbutoxy, 3-Chlorbutoxy, 4-Chlorbutoxy, 1-Fluorbutoxy, 2-Fluorbutoxy, 3-Fluorbutoxy, 4-Fluorbutoxy und dergleichen, bevorzugt sind Fluormethoxy, Difluormethoxy oder Trifluormethoxy.
Der Begriff "Alkylthio" oder "Thioalkyl" bezeichnet geradkettige oder verzweigte gesättigte Alkylgruppen, umfassend 1 bis 10 Kohlenstoffatome, die über ein Schwefelatom gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy.
Der Begriff "Haloalkylthio" beschreibt geradkettige oder verzweigte gesättigte Haloalkylgruppen, umfassend 1 bis 10 Kohlenstoffatome, die über ein Schwefelatom gebunden sind.
Der Begriff "Cycloalkoxy" bezeichnet einen gesättigten alicyclischen Rest mit 3 bis 10 Kohlenstoffatomen als Ringglieder, der über ein Sauerstoffatom gebunden ist. Beispiele sind Cyclopropyloxy, Cyclobutyloxy, Cyclopentyloxy, Cyclohexyloxy, Cycloheptyloxy, Cyclooctyloxy, Cyclononyloxy und Cyclodecyloxy. Die Cycloalkylreste können 1, 2 oder 3 Substituenten tragen, die ausgewählt sind unter Alkyl und Halogen. Bevorzugte Cycloalkoxyreste sind Cyclopropyloxy, Cyclobutyloxy, Cyclopentyloxy oder Cyclohexyloxy.
Der Begriff "Alkylcarbonyl" bezeichnet über eine Carbonylgruppe gebundene Alkylreste mit 1 bis 10 Kohlenstoffatomen, . Beispiele hierfür sind Methylcarbonyl (Acetyl), Ethylcarbonyl, Propylcarbonyl, Isopropylcarbonyl, n-Butylcarbonyl, sec-Butylcarbonyl, Isobutylcarbonyl und tert-Butylcarbonyl, bevorzugt Methylcarbonyl oder Ethylcarbonyl.
Der Begriff "Haloalkylcarbonyl" bezeichnet über eine Carbonylgruppe gebundene Haloalkylreste mit 1 bis 10 Kohlenstoffatomen. Beispiele hierfür sind Fluormethylcarbonyl, Difluormethylcarbonyl, Trifluormethylcarbonyl, 1-Fluorethylcarbonyl, 2-Fluorethylcarbonyl, 1,1-Difluorethylcarbonyl, 2,2-Difluorethylcarbonyl, 2,2,2-Trifluorethylcarbonyl, Pentafluorethylcarbonyl und dergleichen, bevorzugt sind Fluormethylcarbonyl, Difluormethylcarbonyl oder Trifluormethylcarbonyl.
Der Begriff "Alkylcarbonyloxy" bezeichnet über eine Carbonyloxygruppe gebundene Alkylreste mit 1 bis 10 Kohlenstoffatomen. Beispiele hierfür sind Methylcarbonyloxy (Acetoxy), Ethylcarbonyloxy, Propylcarbonyloxy und Isopropylcarbonyloxy, bevorzugt Methylcarbonyloxy oder Ethylcarbonyloxy.
Der Begriff "Haloalkylcarbonyloxy " bezeichnet über eine Carbonyloxygruppe gebundene Haloalkylreste mit 1 bis 10 Kohlenstoffatomen. Beispiele hierfür sind Fluormethylcarbonyloxy, Difluormethylcarbonyloxy, Trifluormethylcarbonyloxy, 1-Fluorethylcarbonyloxy, 2-Fluorethylcarbonyloxy, 1,1-Difluorethylcarbonyloxy, 2,2-Difluorethylcarbonyloxy, 2,2,2-Trifluorethylcarbonyloxy, Pentafluorethylcarbonyloxy und dergleichen, bevorzugt sind Fluormethylcarbonyloxy, Difluormethylcarbonyloxy oder Trifluormethylcarbonyloxy.
Der Begriff "Alkenylcarbonyl" bezeichnet über eine Carbonylgruppe gebundene Alkenylreste mit 2 oder 3 bis 6 Kohlenstoffatomen. Beispiele hierfür sind Propen-1-ylcarbonyl, Propen-2-ylcarbonyl (Allylcarbonyl), But-1-en-1-ylcarbonyl, But-1-en-2-ylcarbonyl, But-1-en-3-ylcarbonyl, But-1-en-4-ylcarbonyl, But-2-en-1-ylcarbonyl, But-2-en-2-ylcarbonyl, But-2-en-4-ylcarbonyl, 2-Methylprop-1-en-1-ylcarbonyl, 2-Methylprop-2-en-1-ylcarbonyl und dergleichen, bevorzugt sind Propen-1-ylcarbonyl, Propen-2-ylcarbonyl oder But-1-en-4-ylcarbonyl.
Der Begriff "Alkoxycarbonyl" bezeichnet über eine Carbonylgruppe gebundene Alkoxyreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methoxycarbonyl, Ethoxycarbonyl, Propoxycarbonyl, Isopropoxycarbonyl, n-Butoxycarbonyl, sec-Butoxycarbonyl, Isobutoxycarbonyl und tert-Butoxycarbonyl, bevorzugt sind Methoxycarbonyl, Ethoxycarbonyl, Propoxycarbonyl oder Isopropoxycarbonyl.
Der Begriff "Haloalkoxycarbonyl" bezeichnet über eine Carbonylgruppe gebundene Haloalkoxyreste mit 1 bis 10 Kohlenstoffatomen. Beispiele hierfür sind Fluormethoxycarbonyl, Difluormethoxycarbonyl, Trifluormethoxycarbonyl, 1-Fluorethoxycarbonyl, 2-Fluorethoxycarbonyl, 1,1-Difluorethoxycarbonyl, 2,2-Difluorethoxycarbonyl, 2,2,2-Trifluorethoxycarbonyl, Pentafluorethoxycarbonyl und dergleichen, bevorzugt sind Fluormethoxycarbonyl, Difluormethoxycarbonyl oder Trifluormethoxycarbonyl.
Der Begriff "Alkenyloxycarbonyl" bezeichnet Alkenyloxyreste mit 2 oder 3 bis 8 Kohlenstoffatomen, die über eine Carbonylgruppe gebunden sind. Beispiele hierfür sind Allyloxycarbonyl und Methallyloxycarbonyl, bevorzugt Allyloxycarbonyl.
Der Begriff "Alkylsulfonyl" bezeichnet über eine Sulfonylgruppe (SO₂) gebundene Alkylreste mit 1 bis 10 Kohlenstoffatomen, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Methylsulfonyl, Ethylsulfonyl, Propylsulfonyl, Isopropylsulfonyl, n-Butylsulfonyl, sec-Butylsulfonyl, Isobutylsulfonyl und tert-Butylsulfonyl, bevorzugt Methylsulfonyl, Ethylsulfonyl, Propylsulfonyl oder Isopropylsulfonyl.
Der Begriff "Haloalkylsulfonyl" bezeichnet über eine Sulfonylgruppe (SO₂) gebundene Haloalkylreste mit 1 bis 10 Kohlenstoffatomen. Beispiele hierfür sind Fluormethylsulfonyl, Difluormethylsulfonyl, Trifluormethylsulfonyl, 1-Fluorethylsulfonyl, 2-Fluorethylsulfonyl, 1,1-Difluorethylsulfonyl, 2,2-Difluorethylsulfonyl, 2,2,2-Trifluorethylsulfonyl, Pentafluorethylsulfonyl und dergleichen, bevorzugt Fluormethylsulfonyl, Difluormethylsulfonyl oder Trifluormethylsulfonyl.
Der Begriff "Aryl" bezeichnet carbocyclische aromatische Reste mit 6 bis 14 Kohlenstoffatomen. Beispiele hierfür umfassen Phenyl, Naphthyl, Fluorenyl, Azulenyl, Anthracenyl und Phenanthrenyl. Bevorzugt steht Aryl für Phenyl oder Naphthyl und insbesondere Phenyl.
Der Begriff "Aryloxy" bezeichnet carbocyclische aromatische Reste mit 6 bis 14 Kohlenstoffatomen, die über ein Sauerstoffatom gebunden sind.
Der Begriff "Heteroaryl" bezeichnet aromatische Reste mit 1 bis 4 Heteroatomen, die ausgewählt sind unter O, N, S und SO₂. Beispiele hierfür sind 5- und 6-gliedrige Heteroarylreste mit 1, 2, 3 oder 4 Heteroatomen ausgewählt unter O, N, S und SO₂ wie Pyrrolyl, Furanyl, Thienyl, Pyrazolyl, Imidazolyl, Oxazolyl, Isoxazolyl, Thiazolyl, Isothiazolyl, Triazolyl, Tetrazolyl, Pyridyl, Pyrazinyl, Pyridazinyl, Pyrimidyl oder Triazinyl.
Der Begriff "Arylcarbonyl" bezeichnet Arylreste, die über eine Carbonylgruppe gebunden sind. Beispiele hierfür sind Phenylcarbonyl, 4-Nitrophenylcarbonyl, 2-Methoxyphenylcarbonyl, 4-Chlorphenylcarbonyl, 2,4-Dichlorphenylcarbonyl, 4-Nitrophenylcarbonyl oder Naphthylcarbonyl, bevorzugt Phenylcarbonyl.
Der Begriff "Arylalkyl" bezeichnet Arylreste, die über eine Alkylgruppe gebunden sind, wobei der Alkylrest gegebenenfalls 1, 2 oder 3 Substituenten trägt, die ausgewählt sind unter Halogen, Alkyl, Haloalkyl, Cycloalkyl, Alkoxy, Haloalkoxy. Beispiele hierfür sind Benzyl, 2-Phenylethyl (Phenethyl) und dergleichen, bevorzugt Phenethyl.
Der Begriff "Arylmethoxycarbonyl" bezeichnet Arylmethoxyreste, die über eine Carbonylgruppe gebunden sind. Beispiele hierfür sind Benzyloxycarbonyl oder Fluorenylmethoxycarbonyl.
Der Begriff "Alkylimino" bezeichnet einen Rest der Formel -N=R, der über den Stickstoff gebunden ist, worin R für Alkylen steht, wie =CH₂, =CHCH₃, =CHCH₂CH₃, =C(CH₃)₂, =CHCH₂CH₂CH₃, =C(CH₃)CH₂CH₃ oder =CHCH(CH₃)₂.
Der Begriff "Arylalkylimino" bezeichnet einen Rest der Formel -N=R, der über den Stickstoff gebunden ist, worin R für Aryl-alkylen, wie Benzyliden (R = CH-Phenyl) steht.
Der Begriff "Hydroxyalkyl" bezeichnet einen über eine Alkylgruppe gebunden OH Rest. Bevorzugt sind -CH₂OH, -(CH₂)₂OH oder -(CH₂)₃OH.

Die nachfolgend gemachten Ausführungen zu bevorzugten Ausgestaltungen der erfindungsgemäßen Verfahren, insbesondere zu bevorzugten Ausgestaltungen der Reste der verschiedenen Edukte und Produkte und der Reaktionsbedingungen der erfindungsgemäßen Verfahren, gelten sowohl allein für sich genommen als auch insbesondere in jeder denkbaren Kombination miteinander.

Die hierin beschriebenen Umsetzungen werden in für derartige Reaktionen üblichen Reaktionsgefäßen durchgeführt, wobei die Reaktionsführung sowohl kontinuierlich, semikontinuierlich als auch diskontinuierlich ausgestaltet werden kann. Bevorzugt werden die jeweiligen Reaktionen unter Atmosphärendruck durchgeführt. Die Reaktionen können jedoch auch unter vermindertem (z.B. 0.1 bis 1.0 bar) oder erhöhtem Druck (z. B. 1.0 bis 10 bar) durchgeführt werden.

Insbesondere ist es bevorzugt die bevorzugten Ausführungsformen in jeder beliebigen Kombination miteinander zu kombinieren.

Bevorzugt betrifft die vorliegende Erfindung ein Verfahren zur Herstellung einer Verbindungen der Formel **3** 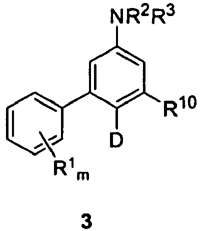 dadurch gekennzeichnet, dass eine Verbindung der Formel **1** 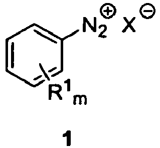 mit einer Verbindung der Formel 2 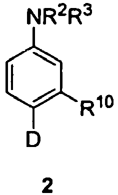 umgesetzt wird, wobei
m für 0, 1, 2, 3, 4 oder 5 steht;
jedes R¹ jeweils unabhängig für Halogen, Alkyl, Haloalkyl, Hydroxyalkyl, Alkoxy, Haloalkoxy, Alkylthio, Cycloalkyl, Haloalkylthio, Alkenyl, Alkinyl, Nitro, Cyano, -SO₃R⁵, -SO₂NH₂, -SO₂NHR⁴, -SO₂NR⁴R⁵, - COOR⁴, -CONHR⁴, -CONR⁴R⁵, -COR⁴, -OCOR⁴, -NR⁴COR⁵, - NR⁴SO₂R⁵, Alkylcarbonyl, Haloalkylcarbonyl, Alkenylcarbonyl, Alykoxycarbonyl, Haloalkoxycarbonyl, Alkenyloxycarbonyl, Alkylsulfonyl, Haloalkylsulfonyl, Alkylimino, Aryl, Aryloxy, Arylcarbonyl, Arylalkyl, Arylmethoxycarbonyl, Arylalkylimino, oder Heteroaryl steht, wobei die Arylgruppe gegebenenfalls 1, 2, 3 oder 4 Substituenten trägt, die ausgewählt sind unter Halogen, Alkyl, Haloalkyl, Alkoxy oder Haloalkoxy;
D für Hydroxy, Alkoxy, Thioalkyl, Haloalkyloxy, Alkylcarbonyloxy, Haloalkylcarbonyloxy oder Aryloxy steht, wobei die Arylgruppe gegebenenfalls 1, 2, 3, 4 oder 5 Substituenten trägt, die ausgewählt sind unter Halogen, Alkyl, Haloalkyl, Alkoxy, Haloalkoxy, Hydroxy, oder Alkoxycabonyl;
X für Halogenid, Sulfat, Tetrafluoroborat, Acetat, Trifluoracetat, Hexafluorophosphat, Hexafluoroantimonat, das Anion eines aromatischen 1,2-Dicarbonsäureimids oder das Anion eines aromatischen 1,2-Disulfonsäureimids steht;
R² jeweils unabhängig für Wasserstoff, Alkyl, Hydroxyalkyl, Cycloalkyl, Aryl, Heteroaryl oder Haloalkyl steht;
R³ jeweils unabhängig für Wasserstoff, Alkyl, Hydroxyalkyl, Cycloalkyl, Aryl, Heteroaryl oder Haloalkyl steht;
R⁴ jeweils unabhängig für Wasserstoff, Alkyl, Hydroxyalkyl, Cycloalkyl, Aryl, Heteroaryl oder Haloalkyl steht;
R⁵ jeweils unabhängig für Wasserstoff, Alkyl, Hydroxyalkyl, Cycloalkyl, Aryl, Heteroaryl oder Haloalkyl steht; und
R¹⁰ jeweils unabhängig für Wasserstoff, Alkyl, Halogen, Hydroxyalkyl, Cycloalkyl, Aryl, Heteroaryl oder Haloalkyl steht.

Im Rahmen der vorliegenden Erfindung steht m bevorzugt für 1, 2, 3 oder 5, insbesondere 1, 2 oder 5. Wenn m für 1 steht, befindet sich R¹ vorzugsweise in para- oder meta-Position zum Diazonium Substituenten.

Im Rahmen der vorliegenden Erfindung steht n bevorzugt für 1, 2, 3 oder 4, insbesondere für 2 oder 3.

Im Rahmen der vorliegenden Erfindung steht q bevorzugt für 1, 2, 3 oder 4, insbesondere für 2 oder 3.

Im Rahmen der vorliegenden Erfindung steht w bevorzugt für 1, 2, 3 oder 4, insbesondere für 2 oder 3.

Im Rahmen der vorliegenden Erfindung steht R¹ bevorzugt für Halogen, Alkyl, Hydroxyalkyl, Haloalkyl, Alkoxy, Haloalkoxy, Nitro, Cyano oder gegebenenfalls mit 1 bis 5 Substituenten, die ausgewählt sind unter Halogen, Alkyl oder Alkoxy substituiertes Aryloxy. Besonders bevorzugt steht R¹ für Halogen, Alkoxy, Haloalkoxy oder gegebenenfalls mit Halogen, Alkyl oder Alkoxy substituiertes Aryloxy und stärker bevorzugt für Chlor, Brom, Fluor, Alkoxy oder Phenoxy. Insbesondere steht R¹ für 2-Me, 3-Me, 4-Me, 2-F, 3-F, 4-F, 2-Cl, 3-Cl, 4-Cl, 2-Br, 3-Br, 4-Br, 2-Methoxy, 3-Methoxy, 4-Methoxy, 2-CF₃, 3-CF₃, 4-CF₄, 2-OCF₃, 3-OCF₃, oder 4-OCF₃. Die Positionsangaben beziehen sich dabei auf die 1-Position, über die der sich von der Verbindung der Formel **1**, bzw. der Formel **8**, bzw. der Formel **10**, bzw. der Formel **12** ableitende Arylrest an den Anilinring der Verbindung der Formel **2**, bzw. der Formel **9**, bzw. der Formel **13** gebunden ist bzw. auf die 1-Position des Diazoniumrestes in der Verbindung der Formel **1**, bzw. der Formel **8**, bzw. der Formel **10**, bzw. der Formel **12.**

Im Rahmen der vorliegenden Erfindung steht X bevorzugt für ein Halogenid, wie Chlorid, Bromid, Iodid, BF₄⁻, PF₆⁻, Sulfat (½ SO₄²⁻), Acetat, das Anion eines aromatischen 1,2-Dicarbonsäureimids oder das Anion eines aromatischen 1,2-Disulfonimids. Das Anion entsteht in den beiden zuletzt genannten Fällen durch Abstraktion des Protons am Imid-Stickstoffatom. Besonders bevorzugt steht X für ein Halogenid, wie Chlorid, Bromid, BF₄⁻oder Sulfat (½ SO₄²⁻).

Im Rahmen der vorliegenden Erfindung steht R² bevorzugt für Wasserstoff, Alkyl, Haloalkyl, Hydroxyalkyl, Cycloalkyl, oder C₂-C₇-Alkyliden (wobei das Stickstoffatom ein Glied eines 3 bis 8-gliedrigen Ringes darstellt).

Im Rahmen der vorliegenden Erfindung steht R³ bevorzugt für Wasserstoff, Alkyl, Haloalkyl, Hydroxyalkyl, Cycloalkyl, oder C₂-C₇-Alkyliden (wobei das Stickstoffatom ein Glied eines 3 bis 8-gliedrigen Ringes darstellt).

Im Rahmen der vorliegenden Erfindung steht R⁴ bevorzugt für Wasserstoff, Alkyl, Haloalkyl, Hydroxyalkyl, Cycloalkyl, oder Aryl, gegebenenfalls mit 1 bis 5 Substituenten, die ausgewählt sind unter Halogen, Alkyl oder Alkoxy substituiertes Aryl.

Im Rahmen der vorliegenden Erfindung steht R⁵ bevorzugt für Wasserstoff, Alkyl, Haloalkyl, Hydroxyalkyl, Cycloalkyl, oder Aryl, gegebenenfalls mit 1 bis 5 Substituenten, die ausgewählt sind unter Halogen, Alkyl oder Alkoxy substituiertes Aryl.

Im Rahmen der vorliegenden Erfindung steht R⁶ bevorzugt für Wasserstoff, Alkyl, Haloalkyl, Hydroxyalkyl, Cycloalkyl, oder Aryl, gegebenenfalls mit 1 bis 5 Substituenten, die ausgewählt sind unter Halogen, Alkyl oder Alkoxy substituiertes Aryl.

Im Rahmen der vorliegenden Erfindung steht R¹⁰ bevorzugt für Wasserstoff, Halogen, Alkyl, Haloalkyl, Alkoxy, Haloalkoxy, Hydroxyalkyl, Cycloalkyl, oder Aryl, gegebenenfalls mit 1 bis 5 Substituenten, die ausgewählt sind unter Halogen, Alkyl oder Alkoxy substituiertes Aryl, besonders bevorzugt Wasserstoff, Halogen oder Alkyl.

Im Rahmen der vorliegenden Erfindung steht R¹³ bevorzugt für Wasserstoff, Halogen, Cyano, Aryl oder Heteroaryl, insbesondere für Wasserstoff, Halogen oder Cyano.

Im Rahmen der vorliegenden Erfindung steht R¹⁷ bevorzugt für Wasserstoff, Halogen, Cyano, Aryl oder Heteroaryl, insbesondere für Wasserstoff, Halogen oder Cyano.

Im Rahmen der vorliegenden Erfindung steht R²² bevorzugt für Wasserstoff, Halogen, Cyano, Aryl oder Heteroaryl, insbesondere für Wasserstoff, Halogen oder Cyano.

Im Rahmen der vorliegenden Erfindung steht R³⁰ bevorzugt für Wasserstoff, Halogen, Cyano, Aryl oder Heteroaryl, insbesondere für Wasserstoff, Halogen oder Cyano.

Im Rahmen der vorliegenden Erfindung steht R³⁵ bevorzugt für Wasserstoff, Halogen, Cyano, Aryl oder Heteroaryl, insbesondere für Wasserstoff, Halogen oder Cyano.

Im Rahmen der vorliegenden Erfindung steht R¹⁴ bevorzugt für Wasserstoff, Alkyl oder Haloalkyl, insbesondere für Wasserstoff.

Im Rahmen der vorliegenden Erfindung steht R¹⁸ bevorzugt für Wasserstoff, Alkyl oder Haloalkyl, insbesondere für Wasserstoff.

Im Rahmen der vorliegenden Erfindung steht R²³ bevorzugt für Wasserstoff, Alkyl oder Haloalkyl, insbesondere für Wasserstoff.

Im Rahmen der vorliegenden Erfindung steht R³¹ bevorzugt für Wasserstoff, Alkyl oder Haloalkyl, insbesondere für Wasserstoff.

Im Rahmen der vorliegenden Erfindung steht R³⁶ bevorzugt für Wasserstoff, Alkyl oder Haloalkyl, insbesondere für Wasserstoff.

Im Rahmen der vorliegenden Erfindung steht R¹⁵ bevorzugt für Wasserstoff, Alkyl, Aryl oder Haloalkyl, insbesondere für Wasserstoff, Alkyl oder Aryl.

Im Rahmen der vorliegenden Erfindung steht R¹⁹ bevorzugt für Wasserstoff, Alkyl, Aryl oder Haloalkyl, insbesondere für Wasserstoff, Alkyl oder Aryl.

Im Rahmen der vorliegenden Erfindung steht R²⁴ bevorzugt für Wasserstoff, Alkyl, Aryl oder Haloalkyl, insbesondere für Wasserstoff, Alkyl oder Aryl.

Im Rahmen der vorliegenden Erfindung steht R³² bevorzugt für Wasserstoff, Alkyl, Aryl oder Haloalkyl, insbesondere für Wasserstoff, Alkyl oder Aryl.

Im Rahmen der vorliegenden Erfindung steht R³⁷ bevorzugt für Wasserstoff, Alkyl, Aryl oder Haloalkyl, insbesondere für Wasserstoff, Alkyl oder Aryl.

Im Rahmen der vorliegenden Erfindung steht D bevorzugt für Hydroxy, Alkoxy, Haloalkoxy, Cycloalkoxy, Alkylcarbonyloxy, Haloalkylcarbonyloxy oder Aryloxy. Für D besonders bevorzugt sind: -OMe, -OCH₂F, -OCHF₂, -OCF₃, -OCF₂Cl, -OEt, -OCF₂CHClF, -OCF₂CHF₂, -O(CH₂)₂Cl, -OCH₂CF₃, -OCF₂CF₃, -O(CH₂)₂CH₃, -OCH(CF₃)₂, -O(CH₂)₂OMe, -OCHMe₂, -O(CH₂)₂CN, -O(CH₂)₃CH₃, -OCMe₃, -OCH(Me)Et, -OCH₂CHMe₂, -O(CH₂)₂OEt, -OCH(Me)CH₂OMe -O(CH₂)₄CH₃, -O(CH₂)₂CHMe₂, -OCHEt₂, -OCH₂CMe₃, -O(CH₂)₂O(CH₂)₂Me, -O(CH₂)₂O(CH₂)₂OMe, -O(CH₂)₅Me, -O(CH₂)₆Me, -OCH(Me)(CH₂)₄Me, -O(CH₂)₇Me, -O(CH₂)₈Me, -O(CH₂)₉Me, -O(CH₂)₁₁Me, -O(CH₂)₁₃Me, -O(CH₂)₁₅Me, -O(CH₂)₁₇Me,

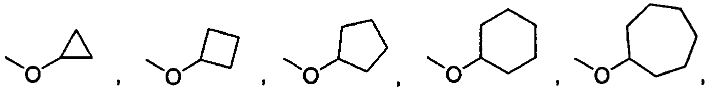

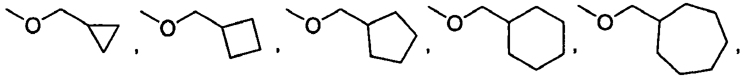

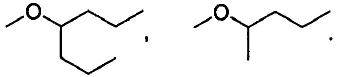

Der Begriff "Reduktionsmittel" bezeichnet diejenigen Elemente und Verbindungen, die als Elektronendonatoren [auch Elektronen-Donator-Komplexe] bestrebt sind, durch die Abgabe von Elektronen in einen energieärmeren Zustand überzugehen, v. a. unter Bildung stabiler Elektronenschalen. Ein Maß für die Stärke eines Reduktionsmittels ist das Redoxpotential. Beispiele für Reduktionsmittel sind anorganische Salze, Metalle, Metallsalze oder reduzierende organische Verbindungen.

Wenn die Reaktion in Gegenwart eines Reduktionsmittels durchgeführt wird, so erfolgt die Durchführung vorzugsweise so, dass die Verbindung der Formel **2,** bzw. der Formel **9**, bzw. der Formel **13** und das Reduktionsmittel, vorzugsweise in einem Lösungsmittel gelöst/dispergiert, vorgelegt und mit der Verbindung der Formel 1, bzw. der Formel **8**, bzw. der Formel **10**, bzw. der Formel **12** sukzessive versetzt werden. Bezüglich Zugabegeschwindigkeit, Reaktionstemperatur und Lösungsmittel wird auf die folgenden Ausführungen verwiesen.

Das wenigstens eine Reduktionsmittel ist vorzugsweise ausgewählt unter reduzierenden Metallsalzen, Metallen und/oder reduzierenden Anionen; geeignet sind jedoch auch andere Reduktionsmittel, deren Reduktionspotential ausreichend groß ist, um auf die jeweils eingesetzte Verbindung der Formel **1**, bzw. der Formel **8**, bzw. der Formel **10,** bzw. der Formel **12** ein Elektron zu übertragen. Dazu gehören so unterschiedliche Verbindungen, wie Pyren, Ascorbinsäure und Hämoglobin. Bevorzugt ist jedoch die Verwendung von reduzierenden Metallen, Metallsalzen und/oder reduzierenden Anionen.

Im Rahmen der vorliegenden Erfindung können beliebige reduzierende Metallsalze verwendet werden, so lange ihr Reduktionspotential ausreichend groß ist, um auf die jeweils eingesetzte Verbindung der Formel **1**, bzw. der Formel **8** bzw. der Formel **10**, bzw. der Formel **12** ein Elektron zu übertragen.
Unter reduzierenden Metallsalzen versteht man im Rahmen der vorliegenden Erfindung solche, in denen unter den Reaktionsbedingungen die stabilste Oxidationszahl des Metalls höher ist als in der eingesetzten Form, so dass das Metallsalz als Reduktionsmittel wirkt.

Bevorzugte Metallsalze sind im Reaktionsmedium löslich. Da das Reaktionsmedium vorzugsweise wässrig ist, sind bevorzugte reduzierende Metallsalze dementsprechend wasserlöslich. Bevorzugte Gegenanionen der Metallsalze sind übliche wasserlösliche Anionen, wie die Halogenide, insbesondere Chlorid, Sulfat, Nitrat, Acetat und dergleichen.

Geeignet sind aber auch Metallkomplexe, wie Hexacyanoferrat(II) oder Ferrocen.

Besonders bevorzugte reduzierende Metallsalze sind ausgewählt unter Ti(III)-Salzen, Cu(I)-Salzen, Fe(II)-Salzen, Zinn(II)-Salzen, Chrom (II)-Salzen und Vanadium(II)-Salzen und insbesondere unter Ti(III)-Salzen, Cu(I)-Salzen und Fe(II)-Salzen. Hierunter bevorzugt sind deren wasserlösliche Salze, wie die Chloride, Sulfate, Nitrate, Acetate und dergleichen.

Insbesondere setzt man Ti(III)-Salze ein und speziell TiCl₃.

Bevorzugte reduzierende Metalle sind ausgewählt unter Eisen, Kupfer, Cobalt, Nickel, Zink, Magnesium, Titan und Chrom, besonders bevorzugt Eisen und Kupfer.

Vorzugsweise setzt man das/die reduzierende(n) Metallsalz(e) in einer Gesamtmenge von 0,005 bis 8 Mol, besonders bevorzugt von 0,1 bis 3 Mol, stärker bevorzugt von 0,1 bis 1 Mol, noch stärker bevorzugt von 0,25 bis 1 Mol und insbesondere von 0,25 bis 0,5 Mol, bezogen auf 1 Mol der Verbindung der Formel **1**, bzw. der Formel **8** bzw. der Formel **10** bzw. der Formel **12.**

Wird die Reaktion in entgasten (d.h. von Sauerstoff befreiten) Lösungsmitteln und unter einer Inertgasatmosphäre, wie Stickstoff oder Argon, durchgeführt, so kann das reduzierende Metallsalz in geringeren Mengen eingesetzt werden, beispielsweise in einer Menge von 0,005 bis 4 Mol, bevorzugt von 0,01 bis 1 Mol, besonders bevorzugt von 0,05 bis 0,7 Mol, stärker bevorzugt 0,05 bis 0,5 Mol und insbesondere von 0,05 bis 0,4 Mol, bezogen auf 1 Mol der Verbindung der Formel **1**, bzw. der Formel **8** bzw. der Formel **10,** bzw. der Formel **12.**

Geeignete reduzierende Anionen sind beispielsweise Bromid, Iodid, Sulfit, Hydrogensulfit, Pyrosulfit, Dithionit, Thiosulfat, Nitrit, Phosphit, Hypophosphit, ArS⁻, Xanthate (R'OCS₂⁻; R' = Alkyl, Aryl), Alkoxide, wie Methanolat, Ethanolat, Propanolat, Isopropanolat, Butanolat, Isobutanolat und tert-Butanolat, und Phenoxid. Wenn die Reaktion unter sauren Bedingungen durchgeführt wird, sind die reduzierenden Anionen selbstverständlich vorzugsweise unter solchen ausgewählt, deren Reduktionspotential auch unter diesen Bedingungen noch ausreicht, um die Zersetzung der Verbindung der Formel **1**, bzw. der Formel **8** bzw. der Formel **10** bzw. der Formel **12** zu bewirken.

Die reduzierenden Anionen werden in einer Menge von vorzugsweise 0,005 bis 8 Mol besonders bevorzugt von 0,01 bis 6 Mol und insbesondere von 1 bis 6 Mol, bezogen auf 1 Mol der Verbindung der Formel **1**, bzw. der Formel **8** bzw. der Formel **10** bzw. der Formel **12** eingesetzt.

Alternativ oder zusätzlich erfolgt in einer bevorzugten Ausführungsform des erfindungsgemäßen Verfahrens die Durchführung unter den Bedingungen einer elektrochemischen Reduktion. Bei dieser Vorgehensweise werden aus der Verbindung der Formel **1**, bzw. der Formel **8** bzw. der Formel **10** bzw. der Formel **12** durch anodische Reduktion Aryldiazenylradikale generiert, was den Zerfall der oben genannten Verbindungen initiiert.

Die Durchführung erfolgt beispielsweise so, dass in das Reaktionsgefäß, welches die in einem geeigneten Lösungsmittel vorgelegte Verbindung der Formel **2** bzw. der Formel **9** enthält, Kathode und Anode angeordnet werden und während der sukzessiven Zugabe der Verbindung der Formel **1**, bzw. der Formel **8** bzw. der Formel **10** bzw. der Formel **12** Spannung angelegt wird. Die zu wählende Spannung und Stromdichte hängt von diversen Faktoren, wie Zugabegeschwindigkeit und Lösungsmittel ab und muss im Einzelfall bestimmt werden, was beispielsweise mit Hilfe von Vorversuchen gelingt. Die Lösungsmittel werden geeigneterweise so gewählt, dass sie unter den gegebenen Reaktionsbedingungen möglichst keine Konkurrenzreaktion an den Elektroden eingehen. Da die anodische Reduktion von Protonen auch bei sehr geringen Stromdichten und Spannung nur schwer zu vermeiden ist, werden bevorzugt nichtprotische, polare Lösungsmittels, wie Acetonitril, Dimethylformamid oder Aceton, eingesetzt.

Von den genannten Maßnahmen ist die Durchführung in Gegenwart wenigstens eines Reduktionsmittels und insbesondere wenigstens eines reduzierenden Metallsalzes oder eines reduzierenden Metalls abhängig von den Reaktionsbedingungen bevorzugt.

Besonders bevorzugt verwendet man jedoch die oben genannten reduzierenden Metallsalze oder Metalle als Reduktionsmittel. Bezüglich geeigneter und bevorzugter Metallsalze wird auf die obigen Ausführungen verwiesen.

Alternativ oder zusätzlich erfolgt das Verfahren zur Herstellung von Verbindungen der Struktur **3**, **5**, **6**, **11** oder **14** dadurch, dass die Reaktion unter Bestrahlung mit elektromagnetischer Strahlung im sichtbaren und/oder ultravioletten Bereich erfolgt. Bevorzugt wird elektromagnetische Strahlung mit einer Wellenlänge im Bereich von 100 bis 400 nm, besonders bevorzugt im Bereich von 200 bis 380 nm und insbesondere im Bereich von 250 bis 360 nm eingesetzt.

Die Durchführung unter Bestrahlung erfolgt bevorzugt in der Weise, dass die Verbindung der Formel **2,** bzw. der Formel **9,** bzw. der Formel **13** in einem geeigneten Lösungsmittel vorgelegt wird und während der sukzessiven Zugabe der Verbindung der Formel **1**, bzw. der Formel **8**, bzw. der Formel **10**, bzw. der Formel **12** unter Kühlen bestrahlt wird. Insbesondere wenn UV-Strahlung verwendet wird, werden die Lösungsmittel vorzugsweise in entgaster Form eingesetzt, da ansonsten Sauerstoffradikale entstehen können, die zu unerwünschten Produkten führen können. Da sich Wasser oder wässrige Lösungen nicht trivial entgasen lassen, bieten sich in diesem Fall die unten genannten organischen Lösungsmittel an.

Alternativ oder zusätzlich erfolgt das Verfahren zur Herstellung von Verbindungen der Formel **3**, **5**, **6**, **11** oder **14** dadurch, dass die Reaktion unter Anwendung von Ultraschall durchgeführt wird. Wie alle Schallwellen erzeugt auch Ultraschall eine periodische Kompression und Dehnung des Mediums; die Moleküle werden zusammengedrückt und gedehnt. Es bilden sich kleine Bläschen, die anwachsen und sofort wieder implodieren. Dieses Phänomen wird Kavitation genannt. Jedes implodierende Bläschen sendet Schockwellen und winzige Flüssigkeitsstrahlen mit einer Geschwindigkeit von etwa 400km/h aus, die auf die nähere Umgebung einwirken. Kavitation kann beispielsweise ausgenutzt werden, um chemische Reaktionen zu beschleunigen und die Löslichkeit von Produkten in einem bestimmten Medium zu erhöhen.

Die Durchführung unter Anwendung von Ultraschall kann beispielsweise so erfolgen, dass sich das Reaktionsgefäß, in welchem die Verbindung der Formel **2,** bzw. der Formel **9,** bzw. der Formel **13** in einem geeigneten Lösungsmittel vorgelegt ist, in einem Ultraschallbad befindet und das Reaktionsgemisch während der sukzessiven Zugabe der Verbindung der Formel **1**, bzw. der Formel **8**, bzw. der Formel **10**, bzw. der Formel **12** Ultraschall ausgesetzt wird. Anstelle der Verwendung eines Ultraschallbads kann in das Reaktionsgefäß, in welchem die Verbindung der Formel **2** bzw. der Formel **9** bzw. der Formel **13** in einem geeigneten Lösungsmittel vorgelegt ist, eine Sonotrode (= Vorrichtung, welche die von einem Schallwandler erzeugten Ultraschallschwingungen an das zu beschallende Material weiterleitet) angebracht werden. Letztere Alternative bietet sich insbesondere für größere Reaktionsansätze an. Bezüglich Zugabegeschwindigkeit, Reaktionstemperatur und Lösungsmittel können gegebenenfalls Vorversuche durchgeführt werden.

Alternativ oder zusätzlich erfolgt in einer bevorzugten Ausführungsform des erfindungsgemäßen Verfahrens die Durchführung unter Radiolysebedingungen. Hierbei werden solvatisierte Elektronen in wässriger Lösung durch Bestrahlung mit γ-Strahlung beispielsweise aus einer ⁶⁰Co-Quelle erzeugt. Diese Verfahrensweise wird näher in J.E. Packer et al., J. Chem. Soc., Perkin Trans. 2, 1975, 751 und im Aust. J. Chem. 1980, 33, 965 beschrieben, worauf hiermit in vollem Umfang Bezug genommen wird.

Der Begriff "Lösungsmittel" (Solvens, Lösemittel) bezeichnet im weitesten Sinne Stoffe, die andere auf phsikalischem Wege zur Lösung bringen können, im engeren Sinne anorganische und organische Flüssigkeiten, die andere gasförmige, flüssige oder feste Stoffe zu lösen vermögen. Voraussetzung für die Eignung als Lösungsmittel ist, dass sich beim Lösungsvorgang weder der lösende noch der gelöste Stofff chemisch verändern, dass also die Komponenten der Lösung durch physikalische Trennverfahren wie Destillation, Kristallisation, Sublimation, Verdunstung, Absorption in der Originalgestalt wiedergewonnen werden können.

Man kennt anorganische und organische Lösungsmittel, die hier gruppenweise vorgestellt werden.
Bei *anorganischen Lösungsmitteln* unterscheidet man Protonen-haltige (protische) wie z.B. H₂O, flüssiges NH₃, H₂S, HF, HCN, HNO₃, und Protonen-(Wasserstoff-)freie (aprotische) Lösungsmittel, wie z.B. flüssiges SO₂, N₂O₄, NOCl, SeOCl₂, ICl, BrF₃, AsCl₃, zum anderen wässrige und nichtwässrige Lösungsmittel (alle außer H₂O, mit den Untergruppen aprotische, amphiprotische, protogene, protophile Lösungsmittel).
Die letzerwähnten Einteilungen lassen sich natürlich erst recht auf die organischen Lösungsmittel anwenden, von denen hier nur die wichtigsten aufgeführt sein können: Alkohole (z.B. Methanol, Ethanol usw.) Glykole (z. B. Ethylenglykol), Ether und Glykolether (z. B. Diethylether, Dioxan, Tetrahydrofuran), Ketone (z.B. Aceton), Amide und andere Stickstoff-Verbindungen (z.B. Dimethylformamid), Schwefelverbindungen (z. B. Schwefelkohlenstoff, Sulfolan), Nitroverbindungen (z. B. Nitromethan), Halogenkohlenwasserstoffe (z. B. Dichlormethan, Chloroform), Kohlenwasserstoffe (z. B. Benzine, Petrolether, Cyclohexan).

Geeignete Lösungsmittel hängen im einzelnen von der Wahl der jeweiligen Reaktionsbedingungen für die Zersetzung der Verbindung der Formel **1**, bzw. der Formel **8**, bzw. der Formel **10**, bzw. der Formel **12**, wie beispielsweise den Reaktionspartnern, ab. Es hat sich jedoch allgemein als günstig erwiesen, als Lösungsmittel für die Umsetzung der Verbindung der Formel **1**, bzw. der Formel **8**, bzw. der Formel **10**, bzw. der Formel **12** und der Verbindung der Formel **2**, bzw. der Formel **9**, bzw. der Formel **13** ein wässriges Lösungsmittel zu verwenden.

Im Rahmen der vorliegenden Erfindung sind bevorzugte organische Lösungsmittel beispielsweise kurzkettige Nitrile, wie Acetonitril oder Propionitril, Amide, wie Dimethylformamid, kurzkettige ein- oder mehrwertige Alkohole, wie Methanol, Ethanol, Propanol, Isopropanol, Ethylenglykol oder Trifluorethanol, kurzkettige Carbonsäuren, wie Eisessig, Trifluoressigsäure, kurzkettige Ketone, wie Aceton, und Dimethylsulfoxid oder Mischungen dieser organischen Lösungsmittel untereinander.

Im Rahmen der vorliegenden Erfindung sind bevorzugte anorganische Lösungsmittel beispielsweise Salzsäure, Schwefelsäure, Phosphorsäure und Flußsäure.

Im Rahmen der erfindungsgemäßen Verfahren wird unter wässrigen Lösungsmitteln Wasser verstanden und unter wässrigen Lösungsmittelsystemen eine Mischung aus Wasser mit wassermischbaren organischen und/oder anorganischen Lösungsmitteln verstanden.

Im Rahmen der vorliegenden Erfindung sind weiterhin wässrige Lösungsmittelsysteme Säurelösungen, insbesondere wässrige Mineralsäuren, wie Salzsäure, Bromwasserstoffsäure, Salpetersäure, Schwefelsäure, Phosphorsäure und dergleichen. Bevorzugt sind hierunter nichtoxidierende Säuren, wie Salzsäure oder Bromwasserstoffsäure. In einer bevorzugten Ausführungsform werden die Säurelösungen dabei in verdünnter Form eingesetzt. "Verdünnt" bedeutet in diesem Zusammenhang, dass die Konzentration der Säure 0,1 bis 20 Gew.-%, insbesondere 3 bis 15 Gew.-% und speziell 2 bis 8 Gew.-%, bezogen auf das Gesamtgewicht des Lösungsmittels beträgt.

Die wässrigen Säurelösungen können auch im Gemisch mit den vorstehend genannten, wassermischbaren organischen Lösungsmitteln eingesetzt werden. In einer besonderen Ausführungsform wird die Konzentration der Säure in dem wässrigen Lösungsmittel so gewählt, dass der pH-Wert des Reaktionsgemisches höchstens 7, z.B. 0 bis 6 oder 1 bis 5 oder 3 bis 5 oder 2 bis 4 beträgt.

In einer bevorzugten Ausführungsform handelt es sich bei dem wässrigem Lösungsmittelsystem um eine verdünnte Mineralsäure, d.h. in dem wässrigem Lösungsmittel liegt eine Mineralsäure in einer Konzentration von in der Regel 0,1 bis 20 Gew.-%, insbesondere von 3 bis 15 Gew.-% und speziell von 2 bis 8 Gew.-% vor. Als Mineralsäure werden hier vorzugsweise Salzsäure oder Schwefelsäure verwendet.

Die Konzentration der Säure in dem wässrigen Lösungsmittelsystem wird vorzugsweise so gewählt, dass im Reaktionsgemisch ein pH-Wert von höchstens 7, z.B. von 0 bis 7, oder 1 bis 7, oder 2 bis 7, und insbesondere von höchstens 5, z.B. von 0 bis 5, oder 1 bis 5, oder 2 bis 5, oder 3 bis 4, vorliegt.

Geeignet sind aber auch nichtwässrige Lösungsmittelsysteme, wie z.B. die o.g. organischen Lösungsmittel und Gemische dieser Lösungsmittel.

Ein weiteres geeignetes Lösungsmittelsystem ist ein Zweiphasen-Lösungsmittelsystem, welches zwei miteinander im Wesentlichen nicht mischbare Lösungsmittelsysteme umfasst. "Im Wesentlichen nicht mischbar" bedeutet, dass sich ein erstes Lösungsmittel, das in geringerer oder gleicher Menge wie ein zweites Lösungsmittel eingesetzt wird, im zweiten Lösungsmittel zu höchstens 20 Gew.-%, vorzugsweise zu höchstens 10 Gew.% und insbesondere zu höchstens 5 Gew.-%, bezogen auf das Gesamtgewicht des ersten Lösungsmittels, löst. Beispiele sind Systeme, die neben einem wie oben definierten wässrigen Lösungsmittel bzw. Lösungsmittelsystem ein oder mehrere mit Wasser im Wesentlichen nicht mischbare Lösungsmittel enthalten, wie Carbonsäureester, z.B. Ethylacetat, Propylacetat oder Ethylpropionat, offenkettige Ether, wie Diethylether, Dipropylether, Dibutylether, Methylisobutylether und Methyl-tert-butylether, aliphatische Kohlenwasserstoffe, wie Pentan, Hexan, Heptan und Octan sowie Petrolether, halogenierte aliphatische Kohlenwasserstoffe, wie Methylenchlorid, Trichlormethan, Dichlorethan und Trichlorethan, cycloaliphatische Kohlenwasserstoffe, wie Cyclopentan und Cyclohexan, und aromatische Kohlenwasserstoffe, wie Toluol, die Xylole, Chlorbenzol, Dichlorbenzole und Mesitylen.

Ein solches Zweiphasen-Lösungsmittelsystem kann geeigneterweise außerdem wenigstens einen Phasentransferkatalysator enthalten.
Geeignete Phasentransferkatalysatoren sind dem Fachmann hinreichend bekannt und umfassen beispielsweise geladene Systeme, wie organische Ammoniumsalze, beispielsweise Tetra-(C₁-C₁₈-alkyl)-ammoniumchloride oder -bromide, wie Tetramethylammoniumchlorid oder -bromid, Tetrabutylammoniumchlorid oder -bromid, Hexadecyltrimethylammoniumchlorid oder -bromid, Octadecyltrimethylammoniumchlorid oder -bromid, Methyltrihexylammoniumchlorid oder -bromid, Methyltrioctylammoniumchlorid oder - bromid oder Benzyltrimethylammoniumhydroxid (Triton B), ferner Tetra-(C₁-C₁₈-alkyl)-phosphoniumchloride oder -bromide, wie Tetraphenylphosphoniumchlorid oder -bromid, [(Phenyl)ₘ-(C₁-C₁₈-alkyl)ₙ]-phosphoniumchloride oder -bromide, worin m = 1 bis 3 und n = 3 bis 1 und die Summe m + n = 4 ist, und außerdem Pyridiniumsalze, wie Methylpyridiniumchlorid oder -bromid, und ungeladene Systeme, wie Kronenether oder Azakronenether, z.B. 12-Krone-4, 15-Krone-5, 18-Krone-6, Dibenzo-18-Krone-6 oder [2,2,2]-Kryptand (222-Kryptofix), Cyclodextrine, Calixarene, wie [1₄]-Metacyclophan, Calix[4]aren und p-tert-Butyl-Calix[4]aren, und Cyclophane.

Geeignet ist auch die Durchführung in einem Zweiphasensystem, wobei eine der Phasen wenigstens eines der oben genannten Lösungsmittel bzw. Lösunsmittelsystem umfasst und die zweite Phase mit der ersten Phase im Wesentlichen nicht mischbar ist. Bevorzugt umfasst die erste Phase wenigstens eines der o.g. protischen Lösungsmittel, wie Wasser, Alkohole oder Diole. Besonders bevorzugt ist die erste Phase ein wässriges System, d.h. als Lösungsmittel wird Wasser, eine wässrige Mineralsäure, wie Salzsäure, Bromwasserstoffsäure, Salpetersäure, Schwefelsäure, Phosphorsäure und dergleichen, oder ein Gemisch aus Wasser oder einer wässrigen Säure mit wenigstens einem mit Wasser mischbaren organischen Lösungsmittel, z.B. Alkohole, wie Methanol, Ethanol, Propanol, Isopropanol oder Trifluorethanol, Diole, wie Ethylenglykol, cyclische Ether, wie Tetrahydrofuran und Dioxan, Acetonitril, Amide, wie Dimethylformamid, Carbonsäuren, wie Eisessig, und Ketone, wie Aceton. Insbesondere umfasst die erste Phase Wasser oder eine wässrige Mineralsäure, wobei die Mineralsäure vorzugsweise Salzsäure oder Bromwasserstoffsäure ist.

Die zweite Phase ist vorzugsweise ausgewählt unter Carbonsäureestern, z.B. Ethylacetat, Propylacetat oder Ethylpropionat, offenkettigen Ethem, wie Diethylether, Dipropylether, Dibutylether, Methylisobutylether und Methyl-tert-butylether, aliphatischen Kohlenwasserstoffen, wie Pentan, Hexan, Heptan und Octan sowie Petrolether, halogenierten aliphatischen Kohlenwasserstoffen, wie Methylenchlorid, Trichlormethan, Tetrachlormethan, Dichlorethan und Trichlorethan, cycloaliphatischen Kohlenwasserstoffen, wie Cyclopentan und Cyclohexan, und aromatischen Kohlenwasserstoffen, wie Toluol, die Xylole, Chlorbenzol oder Dichlorbenzol.

Im Rahmen der vorliegenden Erfindung versteht man unter nichtpolaren (apolaren) Lösungsmitteln beispielsweise Schwefelkohlenstoff, Tetrachlormethan, die meisten aromatischen und die gesättigten aliphatischen Kohlenwasserstoffe, und unter den polaren Lösungsmitteln alle organischen Stickstoff- und Sauerstoff-Verbindungen.
Der Begriff "schwach polare Lösungsmittel" bezeichnet beispielsweise Halogen- und manche aromatischen Kohlenwasserstoffe. Die empirische bestimmte und in Einheiten der sog. E_{T}(30)-Skala ausgedrückte Polarität steigt z.B. von apolarem n-Hexan über Toluol, Chloroform, n-Butanol, Aceton, Ethanol, Formamid bis zum stark polaren Wasser.

Im Rahmen der vorliegenden Erfindung versteht man unter "polaren organischen Lösungsmitteln" beispielsweise kurzkettige Nitrile, wie Acetonitril oder Propionitril, Amide, wie Dimethylformamid, kurzkettige ein- oder mehrwertige Alkohole, wie Methanol, Ethanol, Propanol, Isopropanol, Ethylenglykol oder Trifluorethanol, kurzkettige Carbonsäuren, wie Eisessig, Trifluoressigsäure, kurzkettige Ketone, wie Aceton, und Dimethylsulfoxid.

In einer bevorzugten Ausführungsform werden die Lösungsmittel in entgaster (d.h. speziell in von Sauerstoff befreiter) Form eingesetzt. Das Entgasen von Lösungsmitteln ist bekannt und kann beispielsweise durch ein- oder mehrfaches Einfrieren des Lösungsmittels, Auftauen unter Vakuum (zum Entziehen des im Lösungsmittel gelösten/dispergierten Gases) und Kompensieren mit einem Inertgas, wie Stickstoff oder Argon, erfolgen. Alternativ oder zusätzlich kann das Lösungsmittel mit Ultraschall behandelt werden. Letztere Vorgehensweise bietet sich insbesondere bei Wasser bzw. wässrigen Lösungsmitteln an, da die Ausdehnung von Wasser beim Frieren zu apparativen Problemen führen kann.

Alternativ oder zusätzlich erfolgt in einer bevorzugten Ausführungsform des erfindungsgemäßen Verfahrens die Durchführung in wenigstens einem Lösungsmittel bzw. Lösungsmittelsystem, das die radikalische Zersetzung der Verbindung der Formel **1**, bzw. der Formel **8**, bzw. der Formel **10,** bzw. der Formel **12** in Stickstoff und ein Arylradikal bewirkt und/oder die Umsetzung in einer anderen Art und Weise begünstigt. "In einer anderen Art und Weise begünstigt" bedeutet beispielsweise, dass das Lösungsmittel bzw. Lösungsmittelsystem die Bildung des Arylradikals fördert.

Lösungsmittel bzw. Lösungsmittelsysteme, die die radikalische Zersetzung des Diazoniumsalzes der Formel **1**, bzw. der Formel **8**, bzw. der Formel **10**, bzw. der Formel **12** in ein Arylradikal fördern, zeichnen sich durch ein gewisses reduktives Potential aus und können in Verbindung mit einem Diazoniumsalz der Formel **1**, bzw. der Formel **8**, bzw. der Formel **10**, bzw. der Formel **12** als Reduktionsmittel wirken; d.h. die Lösungsmittel selbst sind oxidierbar. Beispiele für solche Lösungsmittel sind Alkohole, z.B. C₁-C₄-Alkohole, wie Methanol, Ethanol, Propanol, Isopropanol, Butanol, sec-Butanol, Isobutanol und tert-Butanol, Diole, wie Ethylenglykol und Diethylenglykol, offenkettige Ether, wie Diethylether, Methylisobutylether und Methyl-tert-butylether, cyclische Ether, wie Tetrahydrofuran und Dioxan, stickstoffhaltige Heterocyclen, wie Pyridin, und HMPT (Hexamethylphosphorsäuretriamid), aber auch basische wässrige Lösungen. Da allerdings basische Lösungen Nebenreaktionen, wie die Azokupplung des Diazoniumsalzes der Formel 1, bzw. der Formel **8**, bzw. der Formel **10**, bzw. der Formel **12**, fördern oder zumindest nicht unterbinden, sollte der pH des Reaktionsgemischs einen Wert von 9 nicht überschreiten. Geeignete Basen sind anorganische Basen, wie Alkalihydroxide, z.B. Lithium-, Natrium- oder Kaliumhydroxid, Erdalkalihydroxide, z.B. Magnesium- oder Calciumhydroxid, Alkylicarbonate, z.B. Lithium-, Natrium- oder Kaliumcarbonat, und Alkalihydrogencarbonate, z.B. Lithium-, Natrium- oder Kaliumcarbonat, und organische Basen, wie Acetate, z.B. Natriumacetat, oder Alkoholate, z.B. Natriummethanolat, Natriumethanolat, Natrium-tert-Butanolat oder Kalium-tert-Butanolat. Die Alkoholate werden vorzugsweise als wässrige Lösungsmittelsysteme eingesetzt.

Geeignet sind besonders solche Lösungsmittel bzw. Lösungsmittelsysteme, die keine leicht abstrahierbaren Wasserstoffatome besitzen, da sie ein gebildetes Arylradikal bestmöglich vor Nebenreaktionen schützen. Gleichzeitig müssen die Lösungsmittel bzw. Lösungsmittelsysteme aber auch ein ausreichendes Lösungsvermögen für die Reaktanden besitzen. Beispiele hierfür sind Wasser und wässrigen Mineralsäuren, wie Salzsäure, Bromwasserstoffsäure, Salpetersäure, Schwefelsäure, Phosphorsäure und dergleichen, aber auch Alkohole ohne Wasserstoffatome in der α-Position, wie tert-Butanol, oder vergleichsweise inerte organische Lösungsmittel bzw. Lösungsmittelsysteme, wie beispielsweise Acetonitril, Essigsäure, Trifluoressigsäure, Aceton, Trifluorethanol und/oder Dimethylsulfoxid. Insbesondere ein Zusatz von Wasser oder wässrigen Mineralsäuren, wie Salzsäure, Bromwasserstoffsäure, Salpetersäure, Schwefelsäure, Phosphorsäure und dergleichen, wirkt allgemein stabilisierend auf die entstehenden Arylradikale, da diese mit Wasser praktisch keine Nebenreaktionen eingehen. Wasser und wässrige Mineralsäuren sind daher als Lösungsmittel ohne abstrahierbare Wasserstoffatome bevorzugt.

Wenn Lösungsmittel mit leicht abstrahierbaren Wasserstoffatomen, wie primäre Alkohole, eingesetzt werden, so werden sie vorzugsweise im Gemisch mit einem Lösungsmittel, das keine leicht abstrahierbaren Wasserstoffatome besitzt, verwendet. Vorzugsweise werden die dem Arylradikale gegenüber nicht inerten organischen Lösungsmittel bzw. Lösungsmittelsysteme dabei in einer Menge von höchstens 50 Gew.-%, besonders bevorzugt von höchstens 20 Gew.-% und insbesondere von höchstens 10 Gew.-%, bezogen auf das Gewicht des Gemischs aus reduzierenden Lösungsmittel bzw. Lösungsmittelsystem und Lösungsmitteln bzw. Lösungsmittelsystemen ohne leicht abstrahierbare Wasserstoffatome, enthalten. Da als Lösungsmittel bzw. Lösungsmittelsysteme ohne leicht abstrahierbare Wasserstoffatome insbesondere Wasser oder wässrige Mineralsäuren eingesetzt werden, sind die in den Gemischen eingesetzten reduzierenden Lösungsmittel bzw. Lösungsmittelsysteme vorzugsweise solche, die mit Wasser mischbar sind (das sind die zuvor genannten Alkohole, Diole und cyclischen Ether).
Insgesamt werden als Lösungsmittel bzw. Lösungsmittelsysteme, die die radikalische Zersetzung der Verbindung der Formel **1**, bzw. der Formel **8**, bzw. der Formel **10**, bzw. der Formel **12** in Stickstoff und ein Arylradikal bewirken und/oder die Umsetzung in einer anderen Art und Weise begünstigen, vorzugsweise Wasser, die genannten wässrigen Mineralsäuren oder Gemische der oben genannten organischen, mit Wasser mischbaren, ein gewisses Reduktionspotential aufweisenden Lösungsmittel bzw. Lösungsmittelsysteme (das sind die zuvor genannten Alkohole, Diole und cyclischen Ether) mit Wasser oder den genannten wässrigen Säuren eingesetzt.

Alternativ bevorzugt ist die Durchführung in (z. B. alleiniger) Gegenwart wenigstens eines Lösungsmittels, das die radikalische Zersetzung der Verbindung der Formel **1**, bzw. der Formel **8**, bzw. der Formel **10**, bzw. der Formel **12** in Stickstoff und ein Arylradikal bewirkt und/oder die Umsetzung in einer anderen Art und Weise begünstigt, insbesondere von Wasser, einer der oben genannten wässrigen Mineralsäuren oder eines Gemischs der genannten reduktiv wirkenden, organischen, mit Wasser mischbaren Lösungsmittel mit Wasser oder einer der oben genannten wässrigen Mineralsäuren. Bevorzugt werden dabei die Lösungsmittel in entgaster Form eingesetzt. Diese Ausführungsform ist insbesondere dann geeignet, wenn ein Anilin mit einem relativ hohen Reduktionspotential eingesetzt wird, welches als Reduktionsmittel für das Diazoniumsalz der Formel **1**, bzw. der Formel **8**, bzw. der Formel **10**, bzw. der Formel **12** wirkt.

In vielen Fällen verwendet man nicht die reinen Lösungsmittel, sondern Gemische, die die Lösungseigenschaften vereinigen, oder man greift zu Lösungsvermittlern.

Der Begriff "Lösungsvermittler" bezeichnet (grenzflächenaktive) Stoffe, die durch ihre Gegenwart andere, in einem Lösungsmittel praktisch unlösliche Verbindungen in diesem Lösungsmittel löslich oder emulgierbar machen. Es gibt Lösungsvermittler, die mit der schwer löslichen Substanz eine Molekülverbindung eingehen und solche, die durch Micellen-Bildung wirken. Man kann auch sagen , dass Lösungsvermittler einem sog. latenten Lösungsmittel sein Lösungsvermögen verleihen. Beispielsweise ist Ethanol (als latentes Lösungsmittel) löst Celluloseacetat schlecht, nach Zusatz von 2-Nitropropan (als Lösungsvermittler) jedoch viel besser.

Die erfindungsgemäße Umsetzung erfolgt, indem man die Verbindung der Formel **1**, bzw. der Formel **8**, bzw. der Formel **10**, bzw. der Formel **12** und eine Verbindung der Formel **2**, bzw. der Formel **9**, bzw. der Formel **13**, vorzugsweise in einem geeigneten Lösungsmittel, miteinander unter Reaktionsbedingungen in Kontakt bringt, die eine Zersetzung der Verbindung der Formel **1**, bzw. der Formel **8**, bzw. der Formel **10**, bzw. der Formel **12** in Stickstoff und ein Arylradikal bewirken.

Die Reaktanden können prinzipiell in unterschiedlicher Reihenfolge miteinander in Kontakt gebracht werden. So kann beispielsweise die Verbindung der Formel **2,** bzw. der Formel **9** bzw. der Formel **13**, gegebenenfalls in einem Lösungsmittel bzw. Lösungsmittelsystem gelöst oder dispergiert, vorgelegt und mit der Verbindung der Formel 1, bzw. der Formel **8**, bzw. der Formel **10**, bzw. der Formel **12** versetzt werden oder umgekehrt die Verbindung der Formel **1**, bzw. der Formel **8**, bzw. der Formel **10**, bzw. der Formel **12**, gegebenenfalls in einem Lösungsmittel bzw. Lösungsmittelsystem gelöst oder dispergiert, vorgelegt und mit der Verbindung der Formel **2**, bzw. der Formel **9** bzw. der Formel **13** versetzt werden. Es hat sich jedoch allgemein als vorteilhaft erwiesen, die Verbindung der Formel **2**, bzw. der Formel **9** bzw. der Formel **13**, gegebenenfalls in einem Lösungsmittel bzw. Lösungsmittelsystem, vorzulegen und die Verbindung der Formel **1**, bzw. der Formel **8**, bzw. der Formel **10**, bzw. der Formel **12** hinzuzugeben.

Die Umsetzung kann sowohl in einem Lösungsmittel bzw. Lösungsmittelsystem als auch in Substanz durchgeführt werden. In letzterem Fall fungiert beispielsweise die Verbindung der Formel **2**, bzw. der Formel **9** bzw. der Formel **13** selbst als Lösungs- oder Dispergiermittel oder wird, falls sein Schmelzpunkt oberhalb Raumtemperatur (25°C) liegt, als Schmelze vorgelegt und dann mit der Verbindung der Formel **1**, bzw. der Formel **8,** bzw. der Formel **10,** bzw. der Formel **12** unter geeigneten Reaktionsbedingungen versetzt. Die bevorzugte Ausführungsform ist jedoch die Durchführung in einem Lösungsmittel bzw. Lösungsmittelsystem.

Vorzugsweise wird die Verbindung der Formel **2**, bzw. der Formel **9**, bzw. der Formel **13** direkt als freies Amin eingesetzt. Alternativ kann es auch, entweder vollständig oder teilweise, in Form eines seiner Säureaddukte oder einer Mischung solcher Addukte eingesetzt werden, wobei das Hydrochlorid der Verbindung der Formel **2,** bzw. der Formel **9** bzw. der Formel **13** besonders bevorzugt ist.

Die Durchführung erfolgt beispielsweise so, dass die Verbindung der Formel **2,** bzw. der Formel **9**, bzw. der Formel **13** in einem solchen Lösungsmittel(system) vorgelegt und dann das Diazoniumsalz der Formel **1**, bzw. der Formel **8**, bzw. der Formel **10**, bzw. der Formel **12** sukzessive zugegeben wird oder umgekehrt die Verbindung der Formel **1**, bzw. der Formel **8**, bzw. der Formel **10,** bzw. der Formel **12** in einem solchen Lösungsmittel(system) vorgelegt und dann die Verbindung der Formel **2**, bzw. der Formel **9**, bzw. der Formel **13** zugegeben wird, wobei die erste Variante bevorzugt ist. Bezüglich Zugabegeschwindigkeit und Reaktionstemperatur wird auf die obigen Ausführungen verwiesen. Bei Durchführung in einem Zweiphasensystem kann alternativ die Verbindung der Formel **2,** bzw. der Formel **9**, bzw. der Formel **13** im Lösungsmittel(system) der einen Phase und die Verbindung der Formel **1**, bzw. der Formel **8**, bzw. der Formel **10**, bzw. der Formel **12** im Lösungsmittel(system) der zweiten Phase jeweils vorgelegt werden.

Wie bereits gesagt, wird in einer bevorzugten Ausführungsform die Verbindung der Formel **1**, bzw. der Formel **8,** bzw. der Formel **10**, bzw. der Formel **12** zu der in der Vorlage befindlichen Verbindung der Formel **2**, bzw. der Formel **9**, bzw. der Formel **13** gegeben. Die Verbindung der Formel **1**, bzw. der Formel **8**, bzw. der Formel **10,** bzw. der Formel **12** kann sowohl in Substanz als auch in einem Lösungsmittel oder Lösungsmittelsystem gelöst oder dispergiert zugegeben werden. Als Lösungsmittel können hier Wasser oder die oben genannten wässrigen Lösungsmittelsysteme oder polare organische Lösungsmittel eingesetzt werden, wobei die zuvor genannten, wassermischbaren organischen Lösungsmittel bevorzugt sind. Die Verbindung der Formel **1**, bzw. der Formel **8**, bzw. der Formel **10**, bzw. der Formel **12** wird dabei vorzugsweise sukzessive (portionsweise oder kontinuierlich) zugegeben. Die sukzessive Zugabe unterdrückt in vielen Fällen die Bildung von Homokupplungsprodukten, d.h. von Produkten, die durch Reaktion von zwei oder mehreren Verbindungen der Formel **1**, bzw. der Formel **8,** bzw. der Formel **10**, bzw. der Formel **12** miteinander entstehen, denn eine geringe Konzentration der Verbindung der Formel **1**, bzw. der Formel **8**, bzw. der Formel **10**, bzw. der Formel **12** im Reaktionsgemisch gewährleistet, dass seine Umsetzung mit der Verbindung der Formel **2**, bzw. der Formel **9**, bzw. der Formel **13** gegenüber der Umsetzung mit sich selbst überwiegt.

Die Geschwindigkeit der Zugabe wird dabei von mehreren Faktoren bestimmt, wie Ansatzgröße, Temperatur, Reaktivität der Edukte und Art der gewählten Reaktionsbedingungen, die eine Zersetzung der Verbindung der Formel **1**, bzw. der Formel **8**, bzw. der Formel **10**, bzw. der Formel **12** in Stickstoff und ein Arylradikal bewirkt, und kann vom Fachmann im Einzelfall, beispielsweise durch geeignete Vorversuche, bestimmt werden. So verlangt eine geringe Reaktivität der Edukte eine langsamere Zugabegeschwindigkeit, die aber beispielsweise durch eine höhere Temperatur und/oder durch die Wahl von Reaktionsbedingungen, die eine Zersetzung der Verbindung der Formel **1**, bzw. der Formel **8**, bzw. der Formel **10**, bzw. der Formel **12** beschleunigen, zumindest teilweise kompensiert werden kann.

Die beiden zuvor genannten bevorzugten Maßnahmen, d.h. der Einsatz der Verbindung der Formel **1**, bzw. der Formel **8,** bzw. der Formel **10**, bzw. der Formel **12** im Unterschuss sowie deren schrittweise Zugabe, bewirken einen vorteilhaften Reaktionsverlauf, da sie die Homokupplung der Verbindung der Formel **1**, bzw. der Formel **8**, bzw. der Formel **10,** bzw. der Formel **12** zurückdrängen.

Erfindungsgemäß wird die Verbindung der Formel **1**, bzw. der Formel **8**, bzw. der Formel **10,** bzw. der Formel **12** und die Verbindung der Formel **2,** bzw. der Formel **9,** bzw. der Formel **13** unter Reaktionsbedingung umgesetzt, die eine Zersetzung der Verbindung der Formel **1**, bzw. der Formel **8,** bzw. der Formel **10**, bzw. der Formel **12** in Stickstoff und ein Arylradikal bewirken. Dabei werden bevorzugt Reaktionsbedingungen gewählt, unter denen auf das Diazoniumsalz der Formel **1**, bzw. der Formel **8**, bzw. der Formel **10,** bzw. der Formel **12** ein einzelnes Elektron (SET; single electron transfer) übertragen wird.

Geeignete Bedingungen, unter denen eine Zersetzung der Verbindung der Formel **1**, bzw. der Formel **8**, bzw. der Formel **10**, bzw. der Formel **12** in Stickstoff und ein Arylradikal stattfindet, sind dem Fachmann allgemein bekannt. So kann bereits die Zugabe der Verbindung der Formel **1**, bzw. der Formel **8**, bzw. der Formel **10**, bzw. der Formel **12** zu der Verbindung der Formel **2**, bzw. der Formel **9**, bzw. der Formel **13** deren Zersetzung in Stickstoff und einem Arylradikal bewirken, da zumindest ein Teil der Verbindung der Formel **2**, bzw. der Formel **9**, bzw. der Formel **13** ein ausreichendes reduktives Potential besitzt. In diesem Fall müssen keine speziellen Verfahrensmaßnahmen ergriffen werden und die Reaktion kann unter den oben beschriebenen Reaktionsbedingungen durchgeführt werden. Reicht das Reduktionspotential der Verbindung der Formel **2**, bzw. der Formel **9**, bzw. der Formel **13** nicht aus, ist es erforderlich, weitere Verfahrensmaßnahmen zu ergreifen, um den Reduktionsschritt einzuleiten. Aber auch wenn das Reduktionspotential des eingesetzten Anilins ausreichen sollte, um die Zersetzung der Verbindung der Formel **1**, bzw. der Formel **8**, bzw. der Formel **10**, bzw. der Formel **12** in Stickstoff und ein Arylradikal einzuleiten, kann es von Vorteil sein, weitere Verfahrensmaßnahmen zu ergreifen, die einen Zerfall oder Abbau der Verbindung der Formel **1**, bzw. der Formel **8**, bzw. der Formel **10**, bzw. der Formel **12** in Stickstoff und ein Arylradikal gewährleisten/beschleunigen bzw. gegenüber anderen Reaktionsmöglichkeiten des Diazoniumsalzes der Formel **1**, bzw. der Formel **8,** bzw. der Formel **10,** bzw. der Formel **12** (z.B. Azokupplung) bevorzugt ablaufen lassen.

Vorzugsweise sind solche Verfahrensmaßnahmen ausgewählt unter folgenden:
- Durchführung in Gegenwart wenigstens eines Reduktionsmittels;
- Durchführung unter Bestrahlung mit elektromagnetischer Strahlung im sichtbaren und/oder ultravioletten Bereich;
- Durchführung unter Anwendung von Ultraschall;
- Durchführung unter den Bedingungen einer elektrochemischen Reduktion;
- Durchführung in wenigstens einem Lösungsmittel bzw. Lösungsmittelsystem, das die radikalische Zersetzung der Verbindung der Formel **1**, bzw. der Formel **8**, bzw. der Formel **10**, bzw. der Formel **12** in Stickstoff und ein Arylradikal bewirkt und/oder die Umsetzung in einer anderen Art und Weise begünstigt;
- Durchführung unter Radiolysebedingungen;
- und einer Kombination von wenigstens zwei dieser Maßnahmen.

Werden zwei oder mehrere der obigen Maßnahmen kombiniert, so ist vorzugsweise eine dieser Maßnahmen die Durchführung in Gegenwart wenigstens eines Reduktionsmittels und insbesondere wenigstens eines reduzierenden Metallsalzes und/oder die Durchführung in Gegenwart wenigstens eines Lösungsmittels bzw. Lösungsmittelsystems, das die radikalische Zersetzung der Verbindung der Formel **1**, bzw. der Formel **8**, bzw. der Formel **10**, bzw. der Formel **12** in Stickstoff und ein Arylradikal bewirkt und/oder die Umsetzung in einer anderen Art und Weise begünstigt. Geeignet ist insbesondere die Kombination dieser beiden Maßnahmen untereinander sowie mit der Durchführung unter Anwendung von Ultraschall.

Die Reaktionstemperatur wird von mehreren Faktoren bestimmt, wie beispielsweise der Reaktivität der eingesetzten Edukte und der Art der gewählten Reaktionsbedingung, die eine Zersetzung der Verbindung der Formel **1**, bzw. der Formel **8**, bzw. der Formel **10**, bzw. der Formel **12** in Stickstoff und ein Arylradikal bewirkt, und kann vom Fachmann im Einzelfall ermittelt werden, beispielsweise durch einfache Vorversuche. Im Allgemeinen führt man die Umsetzung der Verbindung der Formel **1**, bzw. der Formel **8**, bzw. der Formel **10**, bzw. der Formel **12** mit der Verbindung der Formel **2**, bzw. der Formel **9**, bzw. der Formel **13** bei einer Temperatur im Bereich von vorzugsweise -10 °C bis zum Siedepunkt des Reaktionsgemischs, bevorzugt -78 °C bis 200 °C, besonders bevorzugt von 0 °C bis 80 °C und insbesondere von 0°C bis 50 °C durch. Diese Temperaturen gelten für die Durchführung in Lösung; wird er hingegen in Substanz durchgeführt und liegt der Schmelzpunkt der Verbindung der Formel **2,** bzw. der Formel **9**, bzw. der Formel **13** über Raumtemperatur, entspricht die Reaktionstemperatur selbstverständlich mindestens der Temperatur der Schmelze des Reaktionsgemischs.

Vorzugsweise setzt man in dem erfindungsgemäßen Verfahren zur Herstellung von Verbindungen der Formel **3, 5, 6, 11** bzw. **14** die Verbindung der Formel **1**, bzw. der Formel **8,** bzw. der Formel **10,** bzw. der Formel **12** in einer Menge von 0,001 bis 0,9 Mol, besonders bevorzugt von 0,1 bis 0,7 Mol und insbesondere von 0,1 bis 0,5 Mol, jeweils bezogen auf 1 Mol der Verbindung der Formel **2**, bzw. der Formel **9**, bzw. der Formel **13**, ein.

Diazoniumsalze der Formel **1**, bzw. der Formel **8**, bzw. der Formel **10**, bzw. der Formel **12** sind allgemein bekannt und können gemäß gängiger Verfahren hergestellt werden, wie sie beispielsweise in Organikum, Wiley VCH, 22. Auflage beschrieben sind. So sind sie durch Diazotierung des entsprechenden Anilinderivates erhältlich, beispielsweise, indem man ein solches Anilinderivat mit Nitrit in Gegenwart einer Säure, wie etwa halbkonzentrierte Schwefelsäure, umsetzt. Sowohl entsprechende Anilinderivate zur Herstellung von Verbindungen der Formel **1**, bzw. der Formel **8,** bzw. der Formel **10,** bzw. der Formel **12** als auch von Verbindungen der Formel **2,** bzw. der Formel **9**, bzw. der Formel **13** sind bekannt oder können nach bekannten Verfahren hergestellt werden, beispielsweise, indem man entsprechend substituierte Nitrobenzole in Gegenwart eines geeigneten Katalysators hydriert oder homogen reduziert (etwa mit Sn(II)-Chlorid / HCl, vgl. Houben Weyl, "Methoden d. org. Chemie" 11/1, 422). Auch die Herstellung aus Azobenzolen und die Substitution geeigneter Benzole mit Ammoniak sind gängige Methoden. Die Herstellung von Diazoniumsalzen der Formel **1**, bzw. der Formel **8**, bzw. der Formel **10**, bzw. der Formel **12**, bei denen die Gegenanionen unter den Anionen von aromatischen Dicarbonsäureimiden oder Disulfonimiden ausgewählt sind, kann analog zu M. Barbero et al., Synthesis 1998, 1171-1175 erfolgen.

Die Aufarbeitung der erhaltenen Reaktionsgemische und die Isolierung der Verbindungen der Formel **3**, **5**, **6**, **11** bzw. **14** erfolgt in üblicher Weise, beispielsweise durch eine extraktive Aufarbeitung, durch Entfernen des Lösungsmittels, z. B. unter vermindertem Druck, oder durch eine Kombination dieser Maßnahmen. Eine weitere Reinigung kann beispielsweise durch Kristallisation, Destillation oder durch Chromatographie erfolgen.

Überschüssige bzw. nicht umgesetzte Edukte (hierbei handelt es sich vor allem um die Verbindung der Formel **2**, bzw. der Formel **9**, bzw. der Formel **13**, die ja in Bezug auf die Verbindung der Formel **1**, bzw. der Formel **8**, bzw. der Formel **10**, bzw. der Formel **12** bevorzugt im Überschuss eingesetzt wird) werden bei der Aufarbeitung vorzugsweise isoliert und erneut verwendet.

Entsprechend einer bevorzugten Ausführungsform der Erfindung wird das Reaktionsgemisch zur Aufarbeitung mit einem geeigneten, im Wesentlichen nicht wassermischbaren organischen Lösungsmittel mehrmals extrahiert und die kombinierten organischen Phasen eingeengt. Beispiele für geeignete, im Wesentlichen nicht wassermischbare organische Lösungsmittel sind oben aufgeführt. Das so isolierte Produkt kann anschließend für Verwendungen bereitgehalten oder direkt einer Verwendung zugeführt werden, beispielsweise in einem weiteren Reaktionsschritt eingesetzt werden, oder zuvor weiter aufgereinigt werden.

Insbesondere wenn die Umsetzung in saurer Lösung erfolgt ist, wird vorzugsweise vor der Extraktion mit einem organischen Lösungsmittel das Reaktionsgemisch zumindest teilweise neutralisiert, was in der Regel durch Zugabe einer Base erfolgt. Geeignete Basen sind beispielsweise anorganische Basen, wie Alkalimetallhydroxide, z.B. Lithium-, Natrium- oder Kaliumhydroxid, Erdalkalimetallhydroxide, z.B. Magnesium- oder Calciumhydroxid, oder Alkali- und Erdalkalimetalloxide, z.B. Natrium-, Magnesium- oder Calciumoxid; organische Basen, wie Alkoholate, z.B. Natriummethanolat, Natriumethanolat, Kalium-tert-butanolat und dergleichen; und Amine, wie Diethylamin, Triethylamin oder Ethyldiisopropylamin. Bevorzugt sind die genannten anorganischen Basen, die vorzugsweise als wässrige Lösung verwendet werden, und insbesondere die genannten Alkalimetallhydroxide, wie Lithium-, Natrium- oder Kaliumhydroxid, vorzugsweise in Form ihrer wässrigen Lösung.

### Beispiele

1. Herstellung der Diazoniumssalzlösung (am Beispiel von 4-Chlorphenyldiazoniumchlorid) Zu einer Lösung von fein gepulvertem 4-Chloranilin (2.55 g, 20.0 mmol) in Wasser (20 ml) und 10%iger Salzsäure (20 ml, ca. 3M) (Lösungsmittelgemisch am Rotationsverdampfer dreimal evakuiert und mit Argon belüftet) tropft man bei 0°C unter Argon eine Lösung von Natriumnitrit (1.38 g, 20.0 mmol) in Wasser (10 ml) (zuvor mit Argon entgast) über 10 Minuten zu. Die klare Diazoniumsalzlösung wird weitere 15 Minuten bei 0°C gerührt und unter Argon gehalten. Für den folgenden Versuch werden 5 ml Diazoniumsalzlösung entnommen (5 ml Salzlösung enthalten ca. 2 mmol Diazoniumsalz und 2 mmol Salzsäure).
2. Biarylkupplung
   5 ml der zuvor hergestellten 0.4M Aryldiazoniumchloridlösung (2 mmol) werden mittels Spritzenpumpe tropfenweise unter Argon über 5 Minuten zu einer Lösung des Anilins (10.0 mmol) in Wasser (16 ml, zuvor entgast) und Titan(III)-chlorid (4 ml, ca. 4 mmol, ca. 1M Lösung in 3N Salzsäure) gegeben. Nach beendeter Zugabe wird die Reaktionsmischung weitere 10 Minuten bei Raumtemperatur gerührt und anschließend mit einer Lösung von Natriumhydroxid (2 g) und Natriumsulfit (2 g) in 20 ml Wasser neutralisiert (ca. pH = 8-9). Nach dreimaliger Extraktion mit Ethylacetat (3x50 ml) werden die vereinigten organischen Phasen mit ges. Natriumchloridlösung gewaschen und über Natriumsulfat getrocknet. Die reinen Biarylamine erhält man nach Einengen im Vakuum durch Säulenchromatographie an Kieselgel oder durch Kristallisation.
2.1. 4'-Chlor-6-methoxybiphenyl-3-amin 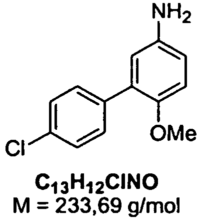 Ausbeute: 55%
   *R_{f}:* 0.5 (1:20 EtOAc-CH₂Cl₂)
   ¹H-NMR: (360 MHz, CDCl₃): δ = 3.44 (br s, 2H), 3.72 (s, 3H), 6.66-6.69 (m, 2H), 6.81-6.84 (m, 1H), 7.36 (d, *J* = 8.8 Hz, 2H), 7.46 (d, *J* = 8.8 Hz, 2H).
   ¹³C-NMR: (90.6 MHz, CDCl₃): δ = 56.3 (CH₃), 113.2 (CH), 115.3(CH), 117.9 (CH), 128.1 (2xCH), 130.3 (Cq), 130.7 (2×CH), 132.8 (Cq), 136.9 (Cq), 140.2 (Cq), 149.6 (Cq).
   EI-MS: *m*/*z* (%) = 235 (21) [³⁷Cl-M⁺], 234 (8), 233 (60) [³⁵Cl-M⁺], 220 (8), 219 (6), 218 (27), 184 (14), 183 (100), 154 (5), 142 (4), 127 (8), 86 (32), 84 (48).
   HR-EI-MS: C₁₃H₁₂³⁵ClNO ber.: 233.0608, gef.: 233.0603.
2.2. 5-Amino-4'-chlorbiphenyl-2-ol 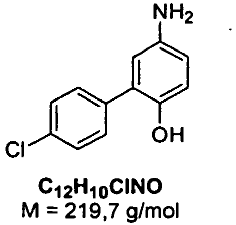
   Ausbeute: 57%
   R_{f}: 0.70(100% EtOAc)
   Schmelzpunkt: 188 °C (Kristallisation aus Dichlormethan)
   ¹H-NMR: (360 MHz, (CD₃)₂SO): δ = 4.52 (s, 2H), 6.45 (dd, *J* = 2.7 Hz, *J* = 8.4 Hz, 1H), 6.54 (d, *J* = 2.7 Hz, 1H), 6.67 (d, *J* = 8.4 Hz, 1H), 7.41 (d, *J* = 8.7 Hz, 2H), 7.52 (d, *J* = 8.7 Hz, 2H), 8.56 (s, 1H).
   ¹³C-NMR: (90.6 MHz, (CD₃)₂SO): δ = 114.9 (CH), 115.6 (CH), 116.8 (CH), 126.4 (Cq), 127.7 (2×CH), 130.6 (2×CH), 130.8 (Cq), 138.0 (Cq), 141.2 (Cq), 145.0 (C_{q}).
   EI-MS: *m*/*z* (%) = 221 (32) [³⁷Cl-M⁺], 220 (12) 219 (100) [³⁵Cl-M⁺], 185 (9), 184 (45), 184 (21), 167 (8), 156 (20), 149 (8), 128 (10), 127 (10), 119 (11), 92 (25), 77 (16), 71 (16).
   HR-EI-MS: C₁₂H_{1O}N₂O³⁷Cl ber.: 221.0421, gef.: 221.0420.
3. Synthese von Tebuquin
3.1. 4'-Chlor-5-(7-chlorchinolin-4-ylamino)biphenyl-2-ol 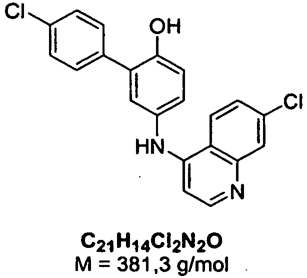 Eine Lösung von 5-Amino-4'-chlorbiphenyl-2-ol (620 mg, 2.82 mmol) und 4,7-Dichlorchinolin (558 mg, 2.82 mmol) in Ethanol (5 ml) und 2N Salzsäure (0.25 ml) wird unter Rückfluß für 4 Stunden erhitzt. Die abgekühlte Lösung wird mit ges. wässrigem Natriumcarbonatlösung (50 ml) versetzt und mit Ethylacetat (5×100 ml) extrahiert. Die vereinigten organischen Phasen mit ges. Natriumchloridlösung gewaschen und über Natriumsulfat getrocknet. Entfernen des Lösungsmittels am Rotationsverdampfer und Trocknen im Vakuum ergibt 4'-Chlor-5-(7-chlorchinolin-4-ylamino)biphenyl-2-ol (935 mg, 2.45 mmol) als gelben Feststoff.
   Ausbeute: 87%
   R_{f}: 0.60 (100% EtOAc)
   Schmelzpunkt: 223 °C (Kristallisation aus Ethylacetat)
   ¹H-NMR: (360 MHz, (CD₃)₂SO): δ = 6.70 (d, *J* = 5.4 Hz, 1H), 7.06 (d, *J* = 8.5 Hz, 1H), 7.19 (dd, *J* = 2.6 Hz, *J* = 8.5 Hz, 1H), 7.26 (d, *J* = 2.6 Hz, 1H), 7.45 (d, *J* = 8.5 Hz, 2H), 7.52 (dd, *J* = 2.2 Hz, *J* = 9.1 Hz, 1H), 7.63 (d, *J* = 8.5 Hz, 2H), 7.86 (d, *J* = 2.2 Hz, 1H), 8.39 (d, *J* = 5.4 Hz, 1H), 8.43 (d, *J* = 9.1 Hz, 1H), 8.96 (s, 1H).
   ¹³C-NMR: (90.6 MHz, (CD₃)₂SO): δ = 100.6 (CH), 117.0 (CH), 117.7 (Cq), 124.1 (CH), 124.5 (CH), 125.1 (CH), 126.2 (CH), 126.9 (Cq), 127.5 (CH), 127.9 (2×CH), 130.7 (2×CH), 131.1 (Cq), 131.4 (Cq), 133.6 (Cq), 136.8 (Cq), 149.2 (Cq), 149.4 (Cq), 151.8 (CH), 151.9 (CH).
   EI-MS: *m*/*z* (%) = 384 (11) [³¹Cl₂-M⁺] 383 (14), 382 (63) [³⁵Cl³⁷Cl-M⁺], 381 (27), 380 (100) [³⁵Cl₂-M⁺], 347 (4), 346 (7), 345 (13), 344 (11), 309 (6).
   HR-EI-MS: C₂₁H₁₄³⁵Cl₂N₂O ber.: 380.0483, gef.: 380.0490.
3.2. [3-*tert*-Butyl-8-(4-chlorphenyl)-3,4-dihydro-2H-benzo[e][1,3]oxazin-6-yl]-(7-chlorchinolin-4-yl)amin 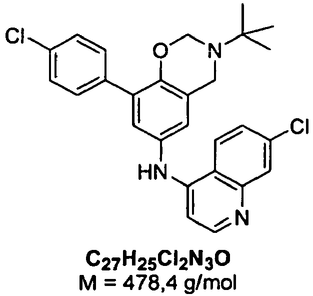 Eine Mischung aus 4'-Chlor-5-(7-chlorchinolin-4-ylamino)biphenyl-2-ol (381 mg, 1.00 mmol), wässrigem Formaldehyd (37%, 4 ml), *tert*-Butylamin (7 ml) und Ethanol (13 ml) wird unter Argon für 5 Stunden unter Rückfluß erhitzt. Der abgekühlte Rückstand wird im Vakuum getrocknet und durch Säulenchromatographie gereinigt (1:20 MeOH-CH₂Cl₂, Kieselgel zuvor mit Triethylamin desaktiviert). Man erhält [3-*tert*-Butyl-8-(4-chlorphenyl)-3,4-dihydro-2H-benzo[*e*][1,3]oxazin-6-yl]-(7-chlor-quinolin-4-yl)amin (448 mg, 0.92 mmol) als gelben Feststoff.
   Ausbeute: 92%
   *R*_{f}: 0.80 (1:20 MeOH-CH₂Cl₂)
   Schmelzpunkt: 220 °C
   ¹H-NMR: (500 MHz, (CD₃)₂SO): δ = 1.17 (s, 9H), 4.17 (s, 2H), 4.98 (s, 2H), 6.79 (d, *J* = 5.4 Hz, 1H), 7.06-7.09 (m, 2H), 7.46 (d, *J* = 8.5 Hz, 2H), 7.54 (dd, *J* = 2.2 Hz, *J* = 8.8 Hz, 1H), 7.55 (d, *J* = 8.5 Hz, 2H), 7.86 (d, *J* = 2.1 Hz, 1H), 8.41 (d, *J* = 5.4 Hz, 1H), 8.42 (d, *J* = 8.8 Hz, 1H), 8.98 (s, 1H).
   ¹³C-NMR: (90.6 MHz, (CD₃)₂SO): δ = 27.9 (3×CH₃), 44.8 (CH₂), 53.7 (Cq), 79.2 (CH₂), 100.8 (CH), 117.8 (Cq), 121.8 (CH), 123.4 (CH), 124.1 (CH), 124.6 (CH), 124.9 (Cq), 127.5 (CH), 127.9 (2xCH), 128.0 (Cq), 130.7 (2×CH), 131.6 (Cq), 131.7 (Cq), 133.6 (Cq), 136.0 (Cq), 148.7 (Cq), 148.9 (Cq), 149.4 (CH), 151.9 (CH).
   EI-MS: *m*/*z* (%) = 479 (8) [³⁵Cl³⁷Cl-M⁺], 478 (4), 477 (12) [³⁵Cl₂-M⁺], 396 (13), 395 (11), 394 (39), 393 (17), 392 (43), 70 (71), 57 (100), 41 (56).
   HR-EI-MS: C₂₇H₂₅³⁵Cl₂N₃O ber.: 477.1375, gef.: 477.1363.
3.3. 3-(*tert*-Butylaminomethyl)-4'-chlor-5-(7-chlorchinolin-4-ylamino)biphenyl-2-ol, Tebuquin (Dihydrochlorid) 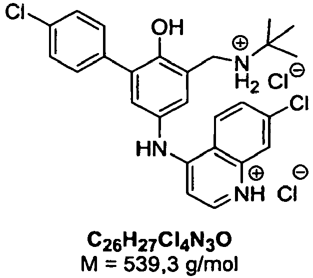 Das Dihydrochlorid von Tebuquin kann aus [3-*tert*-Butyl-8-(4-chlorphenyl)-3,4-dihydro-2H-benzo[*e*][1,3]oxazin-6-yl)-(7-chlor-quinolin-4-yl)amin durch Behandeln mit 3N Salzsäure und anschließendes Trocknen im Vakuum gewonnen werden.
   ¹H-NMR: (360 MHz, CD₃OD): δ = 1.63 (s, 9H), 4.85 (s, 2H), 7.05 (d, *J* = 6.9 Hz, 1H), 7.44 (d, *J* = 8.5 Hz, 2H), 7.49-7.56 (m, 2H), 7.60 (d, *J* = 8.5 Hz, 2H), 7.78 (dd, *J* = 2.0 Hz, *J* = 9.1 Hz, 1H), 8.01 (d, *J* = 2.0 Hz, 1H), 8.45 (d, *J* = 6.9 Hz, 1H), 8.69 (d, *J* = 9.1 Hz, 1H).
   ¹³C-NMR: (90.6 MHz, CD₃OD): δ = 24.8 (3×CH₃), 46.7 (CH₂), 59.0 (Cq), 102.0 (CH), 117.4 (Cq), 120.4 (Cq), 120.5 (CH), 125.7 (CH), 126.6 (CH), 128.4 (CH), 129.3 (CH), 129.6 (2×CH), 132.1 (2×CH), 132.5 (Cq), 133.6 (Cq), 135.3 (Cq), 135.4 (Cq), 140.5 (Cq), 141.5 (Cq), 144.4 (CH), 149.1 (Cq), 157.4 (Cq).
   ESI-MS: *m*/*z* (%) = 470 (5) [³⁷Cl₂-M⁺+H], 469 (7), 468 (28) [³⁵Cl³⁷Cl -M⁺+H], 467 (13), 466 [³⁵Cl₂-M⁺+H], 413 (3), 412 (12), 411 (5); 410 (19), 397 (10), 396 (14), 395 (66), 394 (22), 393 (100).

## Patentansprüche

1. Verfahren zur Herstellung einer Verbindungen der Formel 3, 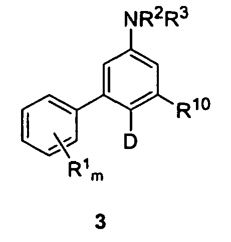 **dadurch gekennzeichnet, dass** eine Verbindung der Formel 1 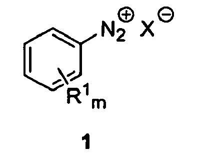 mit einer Verbindung der Formel 2 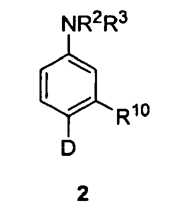 umgesetzt wird, wobei
m für 0, 1, 2, 3, 4 oder 5 steht;
jedes R¹ jeweils unabhängig für Halogen, Alkyl, Haloalkyl, Hydroxyalkyl, Alkoxy, Haloalkoxy, Alkylthio, Cycloalkyl, Haloalkylthio, Alkenyl, Alkinyl, Nitro, Cyano, -SO₃R⁵, -SO₂NH₂, -SO₂NHR⁴, -SO₂NR⁴R⁵, - COOR⁴, -CONHR⁴, -CONR⁴R⁵, -COR⁴, -OCOR⁴, -NR⁴COR⁵, - NR⁴SO₂R⁵, Alkylcarbonyl, Haloalkylcarbonyl, Alkenylcarbonyl, Alkoxycarbonyl, Haloalkoxycarbonyl, Alkenyloxycarbonyl, Alkylsulfonyl, Haloalkylsulfonyl, Alkylimino, Aryl, Aryloxy, Arylcarbonyl, Arylalkyl, Arylmethoxycarbonyl, Arylalkylimino, oder Heteroaryl steht, wobei die Arylgruppe gegebenenfalls 1, 2, 3 oder 4 Substituenten trägt, die ausgewählt sind unter Halogen, Alkyl, Haloalkyl, Alkoxy oder Haloalkoxy;
oder zwei Reste R¹ zusammen eine cyclische Gruppe z.B. der Formel -O-(CR¹¹R¹²)ₜ-O-bilden, wobei t für 1, 2 oder 3 steht, R¹¹ für Wasserstoff, Alkyl oder Haloalkyl steht, R¹² für Wasserstoff, Alkyl, Haloalkyl steht oder ein R¹¹ und ein R¹² zusammen ein nichtaromatisches Ringsystem bilden, welches 5, 6 oder 7 Kohlenstoffatome enthält;
D für Hydroxy, Alkoxy, Thioalkyl, Haloalkyloxy, Alkylcarbonyloxy, Haloalkylcarbonyloxy, -O-(CH₂)ₙ-NR⁴R⁵, -O-(CH₂)ₙ-NHCOR⁴, -O-(CH₂)ₙ-NHC(O)OR⁴, -O-(CH₂)ₙ-COOR⁴, -O-(CH₂)ₙ-CONHR⁴, -O-(CH₂)ₙ-CONR⁴R⁵, -O-(CH₂)ₙ-SO₃R⁴, -O- (CH₂)ₙ-CN oder Aryloxy steht, wobei die Arylgruppe gegebenenfalls 1, 2, 3, 4 oder 5 Substituenten trägt, die ausgewählt sind unter Halogen, Alkyl, Haloalkyl, Alkoxy, Haloalkoxy, Hydroxy, oder Alkoxycarbonyl; oder
in Verbindung 3 einer der Reste R¹ und D zusammen eine Gruppe der Formel -O- oder -C(O)-O- bilden;
X für Halogenid, Sulfat, Tetrafluoroborat, Acetat, Trifluoracetat, Hexafluorophosphat, Hexafluoroantimonat, das Anion eines aromatischen 1,2-Dicarbonsäureimids oder das Anion eines aromatischen 1,2-Disulfonsäureimids steht;
R² jeweils unabhängig für Wasserstoff, Alkyl, Hydroxyalkyl, Cycloalkyl, Aryl, Heteroaryl, -(CH2)ₙ-NR⁴R⁵, -(CH₂)ₙ-NHCOR⁴, -(CH₂)ₙ-NHC(O)O^{R}, - (CH₂)ₙ-COOR⁴, -(CH₂)ₙ-CONHR⁴, -(CH₂)ₙ-CONR⁴R⁵, -(CH₂)ₙ-SO₃R⁴, - (CH₂)ₙ-CN oder Haloalkyl steht;
R³ jeweils unabhängig für Wasserstoff, Alkyl, Hydroxyalkyl, Cycloalkyl, Aryl, Heteroaryl, -(CH₂)ₙ-NR⁴R⁵, -(CH₂)ₙ-NHCOR⁴, -(CH₂)ₙ-NHC(O)OR⁴, - (CH₂)ₙ-COOR⁴, -(CH₂)ₙ-CONHR⁴, -(CH₂)ₙ-CONR⁴R⁵, -(CH₂)ₙ-SO₃R⁴, - (CH₂)ₙ-CN oder Haloalkyl steht;
n jeweils unabhängig für 1, 2, 3, 4, oder 5 steht;
R⁴ jeweils unabhängig für Wasserstoff, Alkyl, Hydroxyalkyl, Cycloalkyl, Aryl, Heteroaryl oder Haloalkyl steht;
R⁵ jeweils unabhängig für Wasserstoff, Alkyl, Hydroxyalkyl, Cycloalkyl, Aryl, Heteroaryl oder Haloalkyl steht;
R¹⁰ jeweils unabhängig für Wasserstoff, Alkyl, Halogen, Hydroxyalkyl, Cycloalkyl, Aryl, Heteroaryl, -(CH₂)_{q}-NR⁴R⁵, -(CH₂)_{q}-NHCOR⁴, - (CH₂)_{q}-NHC(O)OR⁴, -(CH₂)_{q}-COOR⁴, -(CH₂)_{q}-CONHR⁴, -(CH₂)_{q}-CONR⁴R⁵, -(CH₂)_{q}-SO₃R⁴, -(CH₂)_{q}-CN oder Haloalkyl steht; und
q jeweils unabhängig für 1, 2, 3, 4, oder 5 steht.

2. Verfahren nach Anspruch 1, **dadurch gekennzeichnet, dass** D eine Gruppe der Formel - OR⁶ ist, wobei R⁶ für Wasserstoff, Alkyl, Hydroxyalkyl, Alkylcarbonyl, Haloalkylcarbonyl, Cycloalkyl, Aryl, Heteroaryl oder Haloalkyl steht.

3. Verfahren nach Anspruch 1 zur Herstellung einer Verbindung der Formel **7** 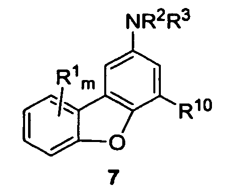 **dadurch gekennzeichnet, dass** in einem ersten Schritt eine Verbindung der Formel **8** mit einer Verbindung der Formel **9** zu einer Verbindung der Formel **6** umgesetzt wird, wobei X¹ für Chlor, Brom oder Iod steht, und in einem zweiten Schritt eine Verbindung der Formel **6** zu der Verbindung der Formel **7** umgesetzt wird:

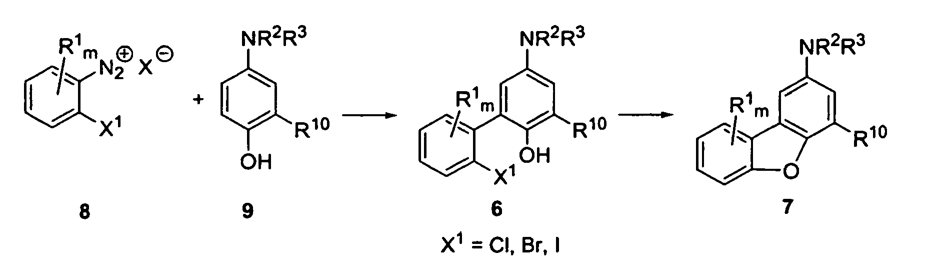

4. Verfahren nach Anspruch 1 zur Herstellung einer Verbindung der Formel **11** 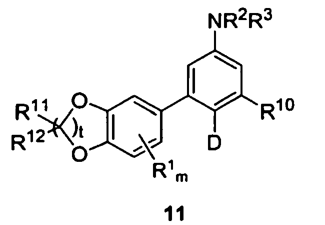 **dadurch gekennzeichnet, dass** eine Verbindung der Formel **10** 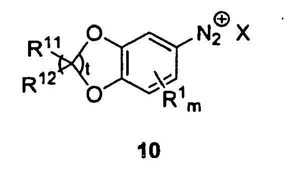 mit einer Verbindung der Formel **2** umgesetzt wird.

5. Verfahren nach Anspruch 1 zur Herstellung einer Verbindung der Formel **14** 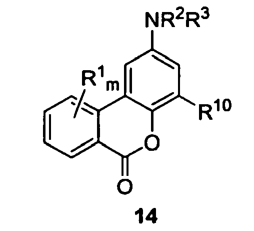 **dadurch gekennzeichnet, dass** eine Verbindung der Formel **12** 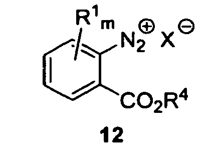 mit einer Verbindung der Formel **13** 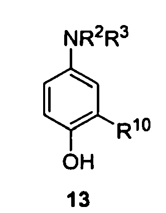 umgesetzt wird.

6. Verfahren nach einem der voranstehenden Ansprüche **dadurch gekennzeichnet, dass** die Umsetzung, bei mehrstufigen Verfahren im ersten Schritt, in Gegenwart mindestens eines Reduktionsmittels durchgeführt wird.

7. Verfahren nach einem der voranstehenden Ansprüche **dadurch gekennzeichnet, dass** die Umsetzung, bei mehrstufigen Verfahren im ersten Schritt, in Gegenwart von mindestens einem Lösungsmittel durchgeführt wird.

8. Verfahren nach einem der voranstehenden Ansprüche **dadurch gekennzeichnet, dass** die Umsetzung, bei mehrstufigen Verfahren im ersten Schritt, unter Bestrahlung, Ultraschall oder Radiolyse durchgeführt wird.

9. Verfahren nach einem der voranstehenden Ansprüche **dadurch gekennzeichnet, dass** die Umsetzung, bei mehrstufigen Verfahren im ersten Schritt, in einem pH-Bereich von 0 bis 11 durchgeführt wird.

10. Verfahren nach einem der voranstehenden Ansprüche **dadurch gekennzeichnet, dass** die Umsetzung, bei mehrstufigen Verfahren im ersten Schritt, unter Schutzgas durchgeführt wird.

11. Verfahren nach einem der voranstehenden Ansprüche **dadurch gekennzeichnet, dass** die Umsetzung, bei mehrstufigen Verfahren im ersten Schritt, im Temperaturbereich von -78°C bis 200°C durchgeführt wird.

## Claims

1. A process for preparing compounds of the formula **3** 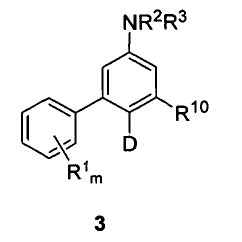 in which a compound of the formula **1** 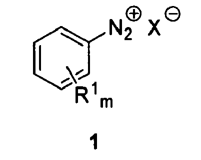 is reacted with a compound of the formula **2** 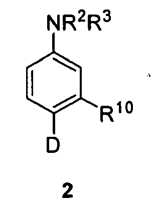 and
m is 0, 1, 2, 3, 4 or 5;
each R¹ is independently halogen, alkyl, haloalkyl, hydroxyalkyl, alkoxy, haloalkoxy, alkylthio, cycloalkyl, haloalkylthio, alkenyl, alkinyl, nitro, cyano, -SO₃R⁵, -SO₂NH₂, -SO₂NHR⁴, -SO₂NR⁴R⁵, -COOR⁴, -CONHR⁴, -CONR⁴R⁵, -COR⁴, -OCOR⁴, -NR⁴COR⁵, -NR⁴SO₂R⁵, Alkylcarbonyl, haloalkylcarbonyl, alkenylcarbonyl, alkoxycarbonyl, haloalkoxycarbonyl, alkenyloxycarbonyl, alkylsulfonyl, haloalkylsulfonyl, alkylimino, aryl, aryloxy, arylcarbonyl, arylalkyl, arylmethoxycarbonyl, arylalkylimino, or heteroaryl, in which aryl optionally bears 1, 2, 3 or 4 substituents, which are selected from the group consisting of halogen, alkyl, haloalkyl, alkoxy or haloalkoxy;
D is hydroxy, alkoxy, thioalkyl, haloalkyloxy, alkylcarbonyloxy, haloalkylcarbonyloxy or aryloxy, in which the aryl group optionally bears 1, 2, 3, 4 or 5 substituents, which are selected from the group halogen, alkyl, haloalkyl, alkoxy, haloalkoxy, hydroxy, or alkoxycabonyl;
X is halogenide, sulfate, tetrafluoroborate, acetate, trifluoracetate, hexafluorophosphate, hexafluoroantimonate, or the anion of an aromatic 1,2-dicarboxylic acid imide or the anion of an aromatic 1,2-disulfonic acid imide;
R² is independently hydrogen, alkyl, hydroxyalkyl, cycloalkyl, aryl, heteroaryl or haloalkyl;
R³ is independently hydrogen, alkyl, hydroxyalkyl, cycloalkyl, aryl, heteroaryl or haloalkyl;
R⁴ is independently hydrogen, alkyl, hydroxyalkyl, cycloalkyl, aryl, heteroaryl or haloalkyl;
R⁵ is independently hydrogen, alkyl, hydroxyalkyl, cycloalkyl, aryl, heteroaryl or haloalkyl;
R¹⁰ is independently hydrogen, alkyl, halogen, hydroxyalkyl, cycloalkyl, aryl, heteroaryl or haloalkyl.

2. A process according to claim 1, in which D denotes a group of the formula -OR⁶, and R⁶ is hydrogen, alkyl, hydroxyalkyl, alkylcarbonyl, haloalkylcarbonyl, cycloalkyl, aryl, heteroaryl or haloalkyl.

3. A process for the preparation of compounds of the formula **7** 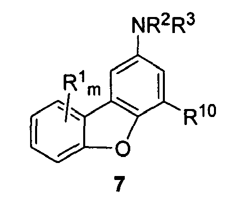 comprising a first step, in which a compound of the formula **8** 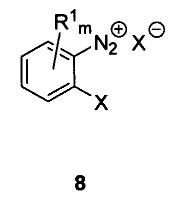 is reacted with a compound of the formula **9** 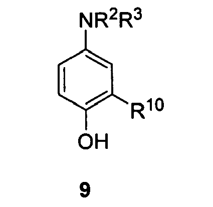 to yield a compound of the formula 6, 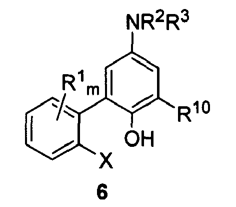 in which
X denotes chlorine, bromine or iodine,
and all other residues are defined as in claim 1,
and a second step, in which the compound of formula **6** is converted to a compound of formula **7.**

4. A process for preparing compounds of the formula **11** 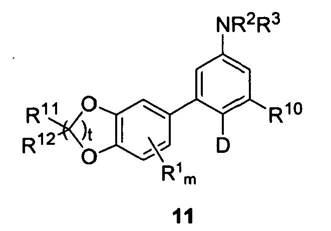 wherein a compound of formula **10** 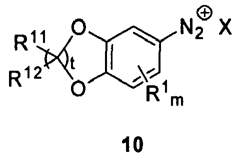 is reacted with a compound of formula **2,** in which
t is 1, 2 or 3;
R¹¹ is hydrogen, alkyl or haloalkyl;
R¹² is hydrogen, alkyl, haloalkyl or
R¹¹ and R¹² forming a non-aromatic ringsystem comprising 5, 6 or 7 carbon atoms,
and all other residues are defined as in claim 1.

5. A process for preparing compounds of the formula **14** 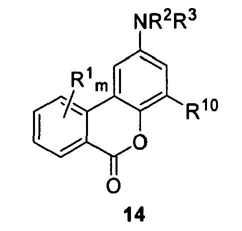 wherein a compound of the formula **12** 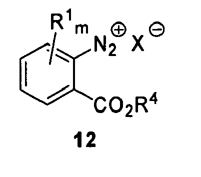 is reacted with a compound of the formula **13** 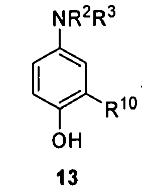 and the residues R¹ to R⁴, R¹⁰, X- and m are defined as above.

6. A process according to one of previous claims denoted by the fact that the first step of a multi-step process is conducted in the presence of at least one reducing agent.

7. A process according to one of previous claims denoted by the fact that the first step of a multi-step process is conducted in the presence of at least one solvent.

8. A process according to one of previous claims denoted by the fact that the first step of a multi-step process is conducted in under irradiation, ultra-sound or radiolysis.

9. A process according to one of previous claims denoted by the fact that the first step of a multi-step process is conducted in a pH range of 0 to 1.

10. A process according to one of previous claims denoted by the fact that the first step of a multi-step process is conducted under protective gas atmosphere.

11. A process according to one of previous claims denoted by the fact that the first step of a multi-step process is conducted in a temperature range of -78°C to 200°C.

## Revendications

1. Procédé de préparation de composés de la formule **3** 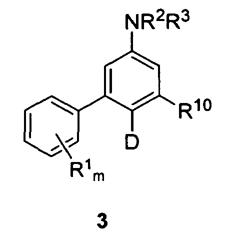 dans laquelle un composé de la formule 1 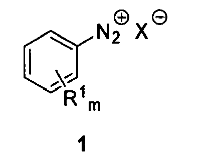 est soumis à une réaction avec un composé de la formule **2** 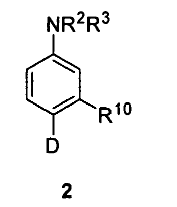 et
m représente 0, 1, 2, 3, 4 ou 5;
chacun R¹ représente indépendamment halogène, alkyle, haloalkyle, hydroxyalkyle, alkoxy, haloalkoxy, alkylthio, cycloalkyl, haloalkylthio, alcényle, alcynyle, nitro, cyano, -SO₃R⁵, -SO₂NH₂, -SO₂NHR⁴, -SO₂NR⁴R⁵, - COOR⁴, -CONHR⁴, -CONR⁴R⁵, -COR⁴, -OCOR⁴, -NR⁴COR⁵, - NR⁴SO₂R⁵, alkylcarbonyle, haloalkylcarbonyle, alcénylcarbonyle, alkoxycarbonyle, haloalkoxycarbonyle, alcényloxycarbonyle, alkylsulfonyle, haloalkylsulfonyle, alkylimino, aryle, aryloxy, arylcarbonyle, arylalkyle, arylmethoxycarbonyle, arylalkylimino, ou heteroaryle, dans lesquelles les groupes aryles sont au choix substitués avec 1, 2, 3 ou 4 substituents selectionnés parmi halogène, alkyle, haloalkyle, alkoxy ou haloalkoxy;
D représente hydroxy, alkoxy, thioalkyle, haloalkyloxy, alkylcarbonyloxy, haloalkylcarbonyloxy ou aryloxy, dans lesquelles les groupes aryles sont au choix substitués avec 1, 2, 3, 4 ou 5 substituents selectionnés parmi halogène, alkyle, haloalkyle, alkoxy, haloalkoxy, hydroxy ou alkoxycarbonyle;
X représente halogénure, sulfate, tetrafluoroborate, acétate, trifluoracétate, hexafluorophosphate, hexafluoroantimonate, ou l'anion d'un 1,2-dicarbonylimide aromatique ou l'anion d'un 1,2-disulfonylimide aromatique;
R² représente indépendamment hydrogène, alkyle, hydroxyalkyle, cycloalkyle, aryle, heteroaryle ou haloalkyle;
R³ représente indépendamment hydrogène, alkyle, hydroxyalkyle, cycloalkyle, aryle, heteroaryle ou haloalkyle;
R⁴ représente indépendamment hydrogène, alkyle, hydroxyalkyle, cycloalkyle, aryle, heteroaryle ou haloalkyle;
R⁵ représente indépendamment hydrogène, alkyle, hydroxyalkyle, cycloalkyle, aryle, heteroaryle ou haloalkyle;
R¹⁰ représente indépendamment hydrogène, alkyle, halogène, hydroxyalkyle, cycloalkyle, aryle, heteroaryle or haloalkyle.

2. Procédé selon la revendication 1, dans lequel D représente un groupe de la formule - OR⁶, et R⁶ représente hydrogène, alkyle, hydroxyalkyle, alkylcarbonyle, haloalkylcarbonyle, cycloalkyle, aryle, heteroaryle or haloalkyle.

3. Procédé de préparation de composés de la formule **7** 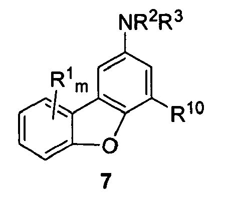 **caractérisé en ce que** dans la permière étape un composé de la formule **8** 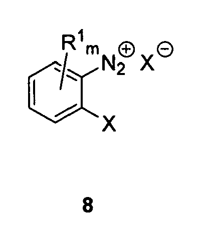 est soumis à une réaction avec un composé de la formule **9** 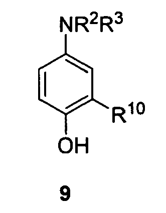 pour obtenir un composé de la formule 6, 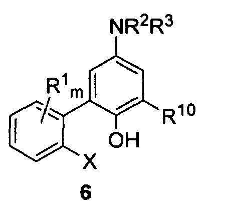 dans laquelle
X représente chlore, brome ou iode,
et toutes autres substituents sont définés selon la revendication 1,
et une deuxième étape, dans laquelle le composé de la formule **6** est transformé dans un composé de la formule **7.**

4. Procédé de préparation de composés de la formule **11** 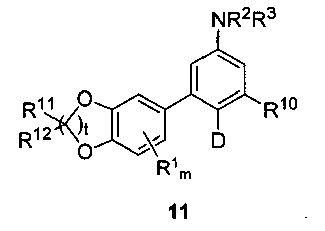 dans laquelle un composé de la formule 10 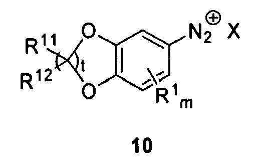 est soumis à une réaction avec un composé de la formule **2,** et
t représente 1, 2 ou 3;
R¹¹ représente hydrogène, alkyle ou haloalkyle;
R¹² représente hydrogène, alkyle, haloalkyle ou
R¹¹ et R¹² composent un système cyclique non-aromatique de 5, 6 ou 7 atomes de carbone,
et toutes autres substituents sont définés selon la revendication 1.

5. Procédé de préparation de composés de la formule **14** 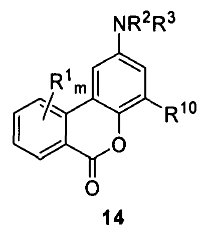 dans laquelle un composé de la formule **12** 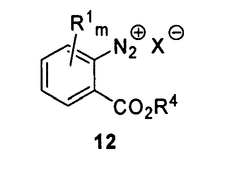 est soumis à une réaction avec un composé de la formule **13** 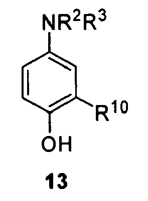 et les substituents R¹ à R⁴, R¹⁰, X- et m sont definès comme dit plus haut.

6. Procédé selon l'une quelconque des revendications précédentes, **caractérisé en ce que** la première étape est effectué en presence de au moins une réducteur.

7. Procédé selon l'une quelconque des revendications précédentes, **caractérisé en ce que** la première étape est effectué en presence de au moins un solvant.

8. Procédé selon l'une quelconque des revendications précédentes, **caractérisé en ce que** la première étape est effectué sous irradiation, ultra-son ou radiolyse.

9. Procédé selon l'une quelconque des revendications précédentes, **caractérisé en ce que** dans la première étape le pH est compris entre 0 et 1.

10. Procédé selon l'une quelconque des revendications précédentes, **caractérisé en ce que** la première étape est effectué sous un gaz protecteur.

11. Procédé selon l'une quelconque des revendications précédentes, **caractérisé en ce que** dans la première étape, la température est comprise entre -78°C et 200°C.
